(19) Europäisches Patentamt
European Patent Office
Office européen des brevets

(11) Veröffentlichungsnummer: **0 149 200**
**A1**

(12) # EUROPÄISCHE PATENTANMELDUNG

(21) Anmeldenummer: 84116009.6

(22) Anmeldetag: 20.12.84

(51) Int. Cl.⁴: C 07 D 487/04
C 07 D 471/04, C 07 D 473/00
A 61 K 31/395, A 61 K 31/415
A 61 K 31/44, A 61 K 31/52
A 61 K 31/50

(30) Priorität: 28.12.83 DE 3347290

(43) Veröffentlichungstag der Anmeldung: 24.07.85 Patentblatt 85/30

(84) Benannte Vertragsstaaten: AT BE CH DE FR GB IT LI LU NL SE

(71) Anmelder: Dr. Karl Thomae GmbH
Postfach 1755
D-7950 Biberach (Riss)(DE)

(72) Erfinder: Austel, Volkhard, Dr.
Kapellenweg 7
D-7950 Biberach 1(DE)

(72) Erfinder: Heider, Joachim. Dr.
Am Hang 3
D-7951 Warthausen 1(DE)

(72) Erfinder: Hauel, Norbert Dr.
Händelstrasse 12
D-7950 Biberach 1(DE)

(72) Erfinder: Reiffen, Manfred. Dr.
Matthias-Erzberger-strasse 40
D-7950 Biberach 1(DE)

(72) Erfinder: Nickl, Josef. Dr
Silcherstrasse 8
D-7950 Biberach 1(DE)

(72) Erfinder: van Meel, Jacobus C.A. Dr.
Amriswilstrasse 7
D-7950 Biberach 1(DE)

(72) Erfinder: Diederen. Willi Dr.
Haldenstrasse 1a
D-7950 Biberach 1(DE)

(54) **Neue 2-Phenyl-imidazole, ihre Herstellung und diese Verbindungen enthaltende Arzneimittel.**

(57) Die Erfindung betrifft neue 2-Phenyl-imidazole der allgemeinen Formel

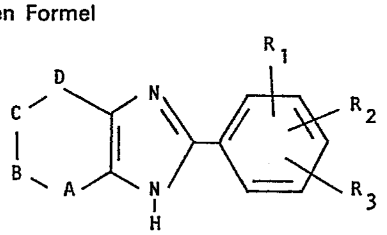

,(I)

in der

A bis D und $R_1$ bis $R_3$ die im Anspruch 1 angegebenen Bedeutungen aufweisen, deren Tautomere und deren Säureadditionssalze, insbesondere deren physiologisch verträgliche Säureadditionssalze, welche wertvolle pharmakologische Eigenschaften aufweisen, insbesondere eine Wirkung auf die Kontraktilität des Herzmuskels.

Die Verbindungen der allgemeinen Formel I können nach für analoge Verbindungen üblichen Verfahren hergestellt werden.

EP 0 149 200 A1

Croydon Printing Company Ltd.

DR. KARL THOMAE GMBH
D-7950 Biberach 1

Case 5/893 0149200
Dr. Fl./Kp.

Neue 2-Phenyl-imidazole, ihre Herstellung und diese Verbindungen enthaltende Arzneimittel

In der EP-A-0.079.083 wird u.a. die Verbindung der Formel

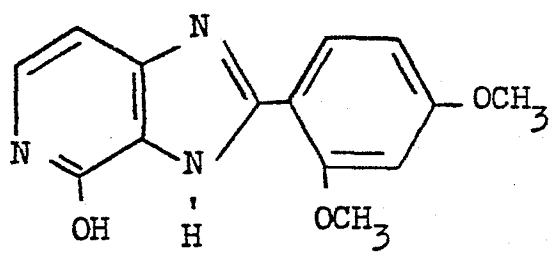

beschrieben, welche positiv inotrope Eigenschaften aufweist.

Es wurde nun gefunden, daß die neuen 2-Phenyl-imidazole der allgemeinen Formel

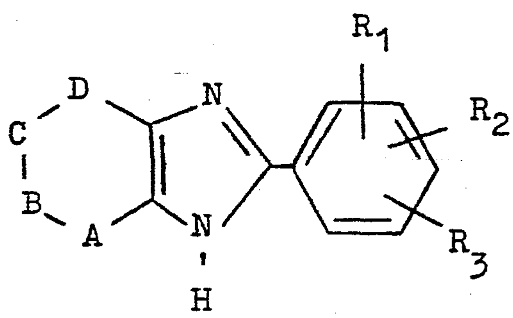

,(I)

deren Tautomere und deren Säureadditionssalze, insbesondere deren physiologisch verträgliche Säureadditionssalze mit anorganischen oder organischen Säuren, welche sich von den

bereits bekannten 2-Phenyl-imidazolderivaten entweder durch den Substituenten $R_1$ oder durch die Reste A, B, C und D unterscheiden, überlegene pharmakologische Eigenschaften aufweisen, insbesondere eine Wirkung auf die Kontraktilität des Herzmuskels.

In der obigen allgemeinen Formel I bedeutet

A, B, C und D je ein gegebenenfalls durch ein Wasserstoffatom oder eine Alkylgruppe mit 1 bis 3 Kohlenstoffatomen substituiertes Stickstoffatom, ein durch ein Wasserstoffatom, ein Halogenatom, eine Hydroxygruppe, eine Benzyloxygruppe oder eine Alkoxygruppe mit 1 bis 3 Kohlenstoffatomen substituiertes Kohlenstoffatom oder eine Carbonylgruppe, wobei jedoch mindestens einer der Reste A, B, C oder D ein gegebenenfalls durch ein Wasserstoffatom oder eine Alkylgruppe substituiertes Stickstoffatom und ein weiterer der Reste A, B, C oder D ein durch ein Halogenatom, eine Hydroxy-, Benzyloxy- oder Alkoxygruppe substituiertes Kohlenstoffatom oder eine Carbonylgruppe darstellen muß,

$R_1$ eine Nitril-, Aminocarbonyl-, Alkylaminocarbonyl-, Dialkylaminocarbonyl-, Alkoxycarbonyl- oder Alkanoylaminogruppe oder,

wenn A, B, C und D zusammen mit dem Rest des Moleküls kein 8-Phenyl-xanthin darstellen, in dem der Phenylring in 2- und/oder 6-Stellung unsubstituiert ist, auch eine Aminosulfonyl-, Alkylaminosulfonyl- oder Dialkylaminosulfonylgruppe oder,

wenn A ein durch ein Wasserstoffatom oder durch eine Alkylgruppe substituiertes Stickstoffatom darstellt, auch eine Alkylmercapto-, Alkylsulfinyl- oder Alkylsulfonylgruppe mit 1 bis 3 Kohlenstoffatomen in jedem Alkylteil, oder,

wenn A ein gegebenenfalls durch ein Wasserstoffatom oder eine Alkylgruppe substituiertes Stickstoffatom darstellt und der Phenylring in 2-Stellung durch eine Alkoxy- oder Dialkylaminogruppe substituiert ist, auch ein Halogenatom, eine Alkyl-, Nitro-, Carboxy-, Amino-, Alkylamino-, Dialkylamino- oder Benzyloxygruppe oder eine 5-ständige Alkoxygruppe oder auch, wenn die Reste A, B, C und D zusammen mit dem Imidazolring keinen Theophyllinrest darstellen, auch eine 4-ständige Alkoxygruppe, wobei der Alkylteil jeweils 1 bis 3 Kohlenstoffatome enthalten kann, oder,

wenn A ein durch ein Wasserstoffatom oder durch eine Alkylgruppe substituiertes Stickstoffatom und $R_2$ und $R_3$ jeweils kein Wasserstoffatom darstellen, auch ein Halogenatom, eine Nitro-, Benzyloxy-, Hydroxysulfonyl- oder eine Alkoxygruppe mit 1 bis 3 Kohlenstoffen im Alkylteil oder,

wenn A und mindestens einer der Reste B und D ein gegebenenfalls durch ein Wasserstoffatom oder eine Alkylgruppe substituiertes Stickstoffatom darstellen, auch ein Halogenatom, eine Alkyl-, Hydroxy-, Benzyloxy-, Alkoxy-, Alkylmercapto-, Alkylsulfinyl-, Alkylsulfonyl-, Hydroxysulfonyl-, Nitro-, Amino-, Alkylamino- oder Dialkylaminogruppe, wobei jeweils der Alkylteil 1 bis 3 Kohlenstoffatome enthalten kann, oder,

wenn B und C jeweils gleichzeitig ein gegebenenfalls durch ein Wasserstoffatom oder eine Alkylgruppe substituiertes Stickstoffatom darstellen, auch ein Halogenatom, eine Alkyl-, Hydroxy-, Benzyloxy-, Alkoxy-, Alkylmercapto-, Alkylsulfinyl-, Alkylsulfonyl-, Hydroxysulfonyl-, Nitro-, Amino-, Alkylamino- oder Dialkylaminogruppe, wobei der Alkylteil jeweils 1 bis 3 Kohlenstoffatome enthalten kann, oder,

wenn A ein Stickstoffatom und C ein durch eine Alkylgruppe substituiertes Stickstoffatom darstellen, auch eine Benzyloxy-, Alkylmercapto-, Alkylsulfinyl- oder Alkylsulfonylgruppe oder, wenn die Reste A, B, C und D zusammen mit dem Imidazolring keinen Xanthinrest darstellen, auch eine Alkyl-, Hydroxy-, Alkoxy-, Nitro-, Amino-, Alkylamino- oder Dialkylaminogruppe, wobei jeweils der Alkylteil 1 bis 3 Kohlenstoffatome enthalten kann, oder

wenn A ein Stickstoffatom und B ein durch eine Alkoxygruppe substituiertes Kohlenstoffatom darstellen, auch ein Halogenatom, eine Alkyl-, Nitro-, Amino-, Alkylamino-, Dialkylamino-, Hydroxy-, Alkoxy-, Benzyloxy-, Alkylmercapto-, Alkylsulfinyl-, Alkylsulfonyl-, Hydroxysulfonyl- oder Carboxygruppe, wobei jeweils jeder Alkylteil 1 bis 3 Kohlenstoffatome enthalten kann,

$R_2$ und $R_3$, die gleich oder verschieden sein können, Wasserstoffatome, Halogenatome, Alkyl-, Hydroxy-, Alkoxy-, Benzyloxy-, Alkylmercapto-, Alkylsulfinyl-, Alkylsulfonyl-, Nitro-, Amino-, Alkylamino-, Dialkylamino-, Alkanoylamino-, Aminosulfonyl-, Alkylaminosulfonyl-, Dialkylaminosulfonyl-, Nitril-, Carboxy-, Alkoxycarbonyl-, Aminocarbonyl-, Alkylaminocarbonyl- oder Dialkylaminocarbonylgruppen, wobei jeweils der Alkylteil 1 bis 3 Kohlenstoffatome enthalten kann.

Gegenstand der vorliegenden Erfindung sind somit die neuen 2-Phenyl-imidazo[4,5-d]pyridazin-4-one, 2-Phenyl-imidazo-[4,5-b]pyridine, 2-Phenyl-imidazo[4,5-b]pyridin-5-one, 6-Hydroxy-2-phenyl-imidazo[4,5-c]pyridin-4-one, 2-Phenyl-imidazo-[4,5-c]-pyridin-4-one, 2-Phenyl-imidazo[4,5-c]pyridin-6-one, 2-Phenyl-imidazo[4,5-d]pyridazin-4,7-dione, 2-Phenyl-imidazo-[4,5-d]pyridazine, 2-Phenyl-imidazo[4,5-c]pyridazin-6-one, 2-Phenyl-imidazo[4,5-c]pyridazine, 8-Phenyl-xanthine, 8-Phenyl-purin-2-one, 8-Phenyl-purin-6-one, 8-Phenyl-purine, 2-Phenyl-imidazo[4,5-c]pyridine, 2-Phenyl-imidazo-[4,5-e]-1,2,4-triazin-6-one, 2-Phenyl-imidazo[4,5-e]-1,2,4-triazine, 2-Phenyl-imidazo[4,5-d]-1,2,3-triazin-7-one, 2-Phenyl-imidazo[4,5-d]-1,2,3-triazine, 2-Phenyl-imidazo[4,5-b]pyrazin-5-one, 2-Phenyl-imidazo[4,5-b]pyrazin-5,6-dione, 2-Phenyl-

imidazo[4,5-b]pyrazine, 2-Phenyl-imidazo[4,5-b]pyridin-7-one, 2-Phenyl-imidazo[4,5-c]pyridazin-7-one und 2-Phenyl-imidazo-[4,5-c]pyridazin-6,7-dione der obigen allgemeinen Formel I, deren Tautomeren, deren Säureadditionssalze, insbesondere deren physiologisch verträgliche Säureadditionssalze mit anorganischen oder organischen Säuren, und diese Verbindungen enthaltende Arzneimittel sowie Verfahren zu ihrer Herstellung.

Für die bei der Definition der Reste A bis D und $R_1$ bis $R_3$ eingangs erwähnten Bedeutungen kommt beispielsweise

für die Reste A bis D jeweils die des Stickstoffatoms, der Imino-, Methylimino-, Ethylimino-, n-Propylimino-, Isopropylimino-, Carbonyl-, Methin-, Chlormethin-, Brommethin-, Hydroxymethin-, Methoxymethin-, Benzyloxymethin-, Ethoxymethin-, n-Propoxymethin- oder Isopropoxymethingruppe und

für die Reste $R_1$ bis $R_3$ jeweils die des Wasserstoff-, Fluor-, Chlor- oder Bromatoms, der Hydroxysulfonyl-, Aminosulfonyl-, Methylaminosulfonyl-, Ethylaminosulfonyl-, n-Propylaminosulfonyl-, Isopropylaminosulfonyl-, Dimethylaminosulfonyl-, Diethylaminosulfonyl-, Di-n-propylaminosulfonyl-, Methyl-ethylaminosulfonyl-, Ethyl-isopropylaminosulfonyl-, Nitril-, Aminocarbonyl-, Methylaminocarbonyl-, Ethylaminocarbonyl-, n-Propylaminocarbonyl-, Isopropylaminocabonyl-, Dimethylaminocarbonyl-, Diethylaminocarbonyl-, Di-n-isopropylaminocarbonyl-, Methyl-ethylaminocarbonyl-, Ethyl-n-propylaminocarbonyl-, Carboxy-, Methoxycarbonyl-, Ethoxycarbonyl-, n-Propoxycarbonyl-, Isopropoxycarbonyl-, Formylamino-, Acetylamino-, Propionylamino-, Methyl-, Ethyl-, n-Propyl-, Isopropyl-, Hydroxy-, Methoxy-, Ethoxy-, n-Propoxy-, Benzyloxy-, Methylmercapto-, Ethylmercapto-, Isopropylmercapto-Methylsulfinyl-, Ethylsulfinyl-, n-Propylsulfinyl-, Methylsulfonyl-, Ethylsulfonyl-, Isopropylsulfonyl-, Nitro-, Amino-, Methylamino-, Ethylamino-, n-Propylamino-, Dimethylamino-, Diethylamino-, Di-n-propylamino-, Ethyl-methylamino- oder Ethyl-n-propylaminogruppe in Betracht.

Bevorzugte Verbindungen der obigen allgemeinen Formel I sind diejenigen, in der

A, B, C und D je ein gegebenenfalls durch ein Wasserstoffatom oder eine Methylgruppe substituiertes Stickstoffatom, ein durch ein Wasserstoff-, Chlor- oder Bromatom, eine Hydroxy-, Methoxy- oder Benzyloxygruppe substituiertes Kohlenstoffatom oder eine Carbonylgruppe, wobei jedoch mindestens einer der Reste A, B , C oder D ein gegebenenfalls durch ein Wasserstoffatom oder eine Methylgruppe substituiertes Stickstoffatom und ein weiterer der Reste A, B, C oder D ein durch ein Chloratom, eine Hydroxy-, Methoxy- oder Benzyloxygruppe substituiertes Kohlenstoffatom oder eine Carbonylgruppe darstellen muß,

$R_1$ eine Nitril-, Aminocarbonyl-, Methylaminocarbonyl-, Dimethylaminocarbonyl-, Methoxycarbonyl- oder Acetylaminogruppe oder,

wenn A, B, C und D zusammen mit dem Rest des Moleküls kein 8-Phenyl-xanthin darstellen, in dem der Phenylring in 2- und/oder 6-Stellung unsubstituiert ist, auch eine Aminosulfonyl-, Methylaminosulfonyl- oder Dimethylaminosulfonylgruppe oder,

wenn A ein durch ein Wasserstoffatom oder eine Methylgruppe substituiertes Stickstoffatom darstellt, auch eine Methylmercapto-, Methylsulfinyl- oder Methylsulfonylgruppe oder, wenn A ein gegebenenfalls durch ein Wasserstoffatom oder eine Methylgruppe substituiertes Stickstoffatom darstellt und der Phenylring in 2-Stellung durch eine Methoxy-, Ethoxy-, Propoxy- oder Dimethylaminogruppe substituiert ist, auch ein Chlor- oder Bromatom, eine Methyl-, Nitro-, Amino-, Methylamino-, Dimethylamino- oder Benzyloxygruppe oder eine 5-ständige Methoxygruppe oder, wenn die Reste A, B, C und D zusammen mit dem Imidazolring keinen Theophyllinrest darstellen, auch eine 4-ständige Methoxygruppe oder,

wenn A ein durch ein Wasserstoffatom oder eine Methylgruppe substituiertes Stickstoffatom und $R_2$ und $R_3$ jeweils kein Wasserstoffatom darstellen, auch ein Chlor- oder Bromatom, eine Nitro-, Hydroxysulfonyl-, Methoxy-, Ethoxy-, Propoxy- oder Benzyloxygruppe oder,

wenn A und mindestens einer der Reste B oder D ein gegebenenfalls durch ein Wasserstoffatom oder eine Methylgruppe substituiertes Stickstoffatom darstellen, auch ein Chloratom, eine Methyl-, Hydroxy-, Benzyloxy-, Methoxy-, Ethoxy-, Propoxy-, Methylmercapto-, Methylsulfinyl-, Methylsulfonyl-, Hydroxysulfonyl-, Amino-, Methylamino- oder Dimethylaminogruppe oder,

wenn B und C jeweils gleichzeitig ein gegebenenfalls durch ein Wasserstoffatom oder eine Methylgruppe substituiertes Stickstoffatom darstellen, auch ein Chloratom, eine Methyl-, Hydroxy-, Methoxy-, Ethoxy-, Propoxy-, Benzyloxy-, Methylmercapto-, Methylsulfinyl-, Methylsulfonyl-, Hydroxysulfonyl-, Nitro-, Amino-, Methylamino- oder Dimethylaminogruppe oder,

wenn A ein Stickstoffatom und C ein durch eine Methylgruppe substituiertes Stickstoffatom darstellen, auch eine Methylmercapto-, Methylsulfinyl- oder Methylsulfonylgruppe oder, wenn die Reste A, B, C und D zusammen mit dem Imidazolring keinen Xanthinrest darstellen, auch eine Methyl-, Hydroxy-, Methoxy-, Ethoxy-, Propoxy-, Nitro-, Amino-, Methylamino- oder Dimethylaminogruppe oder,

wenn A ein Stickstoffatom und B ein durch eine Methoxygruppe substituiertes Kohlenstoffatom darstellen, auch ein Chlor- oder Bromatom, eine Methyl-, Nitro-, Amino-, Methylamino-, Dimethylamino-, Hydroxy-, Methoxy-, Ethoxy-, Propoxy-, Benzyloxy-, Methylmercapto-, Methylsulfinyl-, Methylsulfonyl-, Hydroxysulfonyl- oder Carboxylgruppe,

$R_2$ ein Wasserstoff-, Chlor- oder Bromatom, eine Methyl-, Hydroxy-, Methoxy-, Ethoxy-, Benzyloxy-, Methylmercapto-,

Methylsulfinyl-, Methylsulfonyl-, Nitro-, Amino-, Methylamino-, Dimethylamino-, Acetylamino-, Aminosulfonyl-, Methylaminosulfonyl-, Dimethylaminosulfonyl-, Methoxycarbonyl-, Aminocarbonyl-, Methyl- aminocarbonyl- oder Dimethylaminocarbonylgruppe und

$R_3$ ein Wasserstoffatom, eine Dimethylamino-, Hydroxy-, Methoxy-, Ethoxy- oder Propoxygruppe bedeuten, insbesondere jedoch diejenigen Verbindungen, in denen

$R_1$ in 4- oder 5-Stellung steht und wie vorstehend definiert ist,
$R_3$ in 3-Stellung mit Ausnahme des Wasserstoffatoms die für $R_3$ vorstehend erwähnten Bedeutungen besitzt und
$R_2$ ein Wasserstoff-, Chlor- oder Bromatom, eine Nitro-, Aminosulfonylamino- oder Methylaminosulfonylaminogruppe darstellt,
sowie diejenigen, in denen A, B, C, D, $R_1$ und $R_3$ wie vorstehend definiert sind und
$R_2$ ein Wasserstoffatom darstellt,
besonders bevorzugte Verbindungen sind jedoch diejenigen, in denen $R_1$ in 4-Stellung eine Benzyloxy-, Methylmercapto-, Methylsulfinyl-, Methylsulfonyl-, Aminosulfonyl-, Methylaminosulfonyl- oder Dimethylaminosulfonylgruppe,
$R_3$ in 2-Stellung eine Methoxygruppe und
$R_2$ ein Wasserstoffatom bedeuten, sowie deren Tautomere und deren Säureadditionssalze, insbesondere deren physiologisch verträglichen Säureadditionssalze mit anorganischen oder organischen Säuren.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

in der A bis D wie eingangs definiert sind, einer der Reste X oder Y ein Wasserstoffatom und der andere der beiden Reste X und Y oder beide Reste X und Y eine Gruppe der Formel

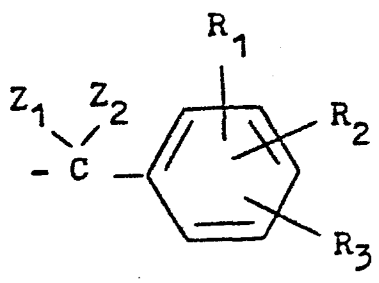

darstellen, in der

$R_1$ bis $R_3$ wie eingangs definiert sind, $Z_1$ und $Z_2$, die gleich oder verschieden sein können, gegebenenfalls substituierte Aminogruppe oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin, Pyridin oder in einem Überschuß des zur Herstellung der Verbindung der allgemeinen Formel II verwendeten Acylierungsmittel, z.B. in dem entsprechenden Nitril, Anhydrid, Säurehalogenid, Ester, Amid oder Methojodid, beispielsweise bei Temperturen zwischen 0 und 250°C,

vorzugszweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Natriumhydroxid, Kaliumhydroxid, Kaliumäthylat oder Kalium-tert.-butylat durchgeführt. Die Cyclisierung kann jedoch auch ohne Lösungsmittel und/oder Kondensationsmittel durchgeführt werden.

b) Zur Herstellung von Verbindungen der allgemeinen Formel I, in der mindestens einer der Reste $R_1$, $R_2$ oder $R_3$ eine Alkylsulfinyl- oder Alkylsulfonylgruppe darstellt:

Oxidation einer Verbindung der allgemeinen Formel

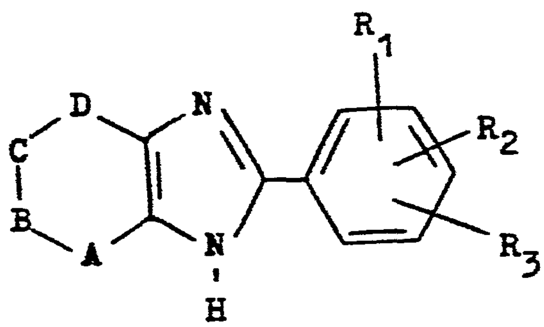

,(III)

in der
A bis D und $R_1$ bis $R_3$ wie eingangs definiert sind, wobei jedoch mindestens einer der Reste $R_1$, $R_2$ oder $R_3$ eine Alkylmercapto- oder Alkylsulfinylgruppe mit jeweils 1 bis 3 Kohlenstoffatomen in jedem Alkylteil darstellen muß.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Alkylsulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig oder wässriger Essigsäure, mit N-Brom-succinimid in Äthanol, mit tert.Butyl-hyprochlorit in Methanol bei -80 bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Alkylsulfonylverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

c) Zur Herstellung von Verbindungen der allgemeinen Formel I, in der mindestens einer der Reste $R_1$, $R_2$ oder $R_3$ eine Hydroxysulfonyl-, Aminosulfonyl-, Alkylaminosulfonyl- oder Dialkylaminosulfonylgruppe darstellt:

Umsetzung einer gegebenenfalls im Reaktionsgemisch gebildeten Verbindung der allgemeinen Formel

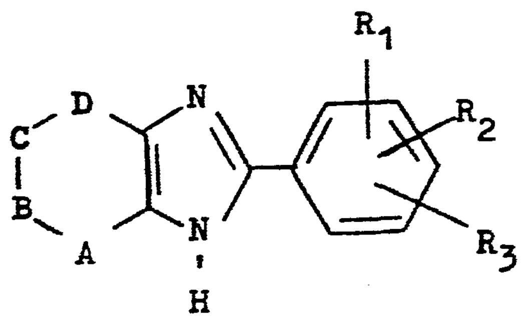

,(IV)

in der

A bis D und $R_1$ bis $R_3$ wie eingangs definiert sind, wobei jedoch mindestens einer der Reste $R_1$, $R_2$ oder $R_3$ eine Gruppe der Formel $USO_2$- darstellen muß, in der U eine nukleophile Austrittsgruppe wie ein Halogenatom darstellt, mit einer Verbindung der allgemeinen Formel

H - V ,(V)

in der

V eine Hydroxygruppe oder eine gegebenenfalls durch eine oder zwei Alkylgruppen mit jeweils 1 bis 3 Kohlenstoffatomen substituierte Aminogruppe darstellt.

Die Umsetzung wird zweckmäßigerweise in einem Lösungsmittel oder Lösungsmittelgemisch wie Wasser, Methanol, Ethanol, Isopropanol, Methylenchlorid, Ether, Tetrahydrofuran, Dioxan, Dimethylformamid oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

d) Zur Herstellung von Verbindungen der allgemeinen Formel I, in der mindestens einer der Reste $R_1$, $R_2$ oder $R_3$

eine durch eine Amino-, Alkylamino- oder Dialkylaminogruppe substituierte Carbonylgruppe darstellt:

Umsetzung einer Verbindung der allgemeinen Formel

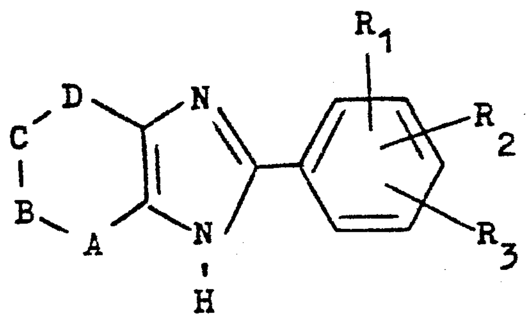

,(VI)

in der
A bis D und $R_1$ bis $R_3$ wie eingangs definiert sind, wobei jedoch mindestens einer der Reste $R_1$, $R_2$ oder $R_3$ die W-CO-Gruppe, in der
W eine Hydroxygruppe oder eine nukleophile Austrittsgruppe wie ein Halogenatom, Aryloxy-, Acyloxy- oder eine Alkoxygruppe darstellt, darstellen muß oder eines reaktionsfähigen Derivates hiervon mit einem Amin der allgemeinen Formel

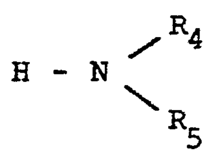

(VII)

in der
$R_4$ und $R_5$, die gleich oder verschieden sein können, Wasserstoffatome oder Alkylgruppen mit 1 bis 3 Kohlenstoffatome darstellen, oder mit einem reaktionsfähigen Derivat hiervon, falls W die Hydroxygruppe darstellt.

Die Umsetzung wird zweckmäßigerweise in einem Lösungsmitel oder Lösungsmittelgemisch wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

e) Zur Herstellung von Verbindungen der allgemeinen Formel I, in der mindestens einer der Reste A, B, C oder D ein durch eine Hydroxy-, Alkoxy- oder Phenylalkoxygruppe substituiertes Kohlenstoffatom oder die Carbonylgruppe darstellt:

Umsetzung einer Verbindung der allgemeinen Formel

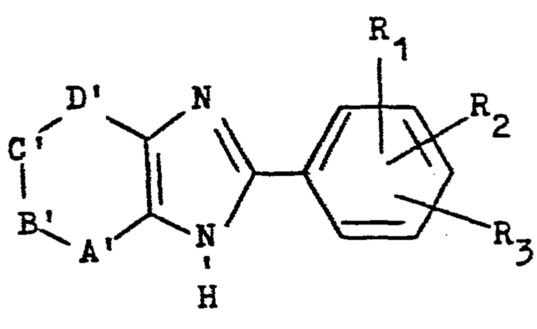

,(VIII)

in der

einer der Reste A', B', C' oder D' ein durch ein Halogenatom substituiertes Kohlenstoffatom darstellt und die übrigen

der Reste A' bis D' die für A bis D eingangs erwähnten Bedeutungen besitzen, mit einer Verbindung der allgemeinen Formel

$$H - R_6 \quad ,(IX)$$

in der

$R_6$ eine Hydroxy-, Alkoxy- oder Phenylalkoxygruppe darstellt, wobei der Alkylteil jeweils 1 bis 3 Kohlenstoffatome enthalten kann.

Die Umsetzung wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid oder dem Alkalisalz eines entsprechenden Alkohols in der Schmelze oder in einem geeigneten Lösungmittel wie Wasser, Wasser/Methanol, Ethanol, Wasser/Ethanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Bei der Umsetzung in der Schmelze, welche zweckmäßigerweise bei Temperaturen bis zu 200°C durchgeführt wird, können gegebenenfalls vorhandene Ethergruppen gleichzeitig in Hydroxygruppen übergeführt werden.

f) Zur Herstellung von Verbindungen der allgemeinen Formel I, in der mindestens einer der Reste A, B, C oder D ein durch eine Hydroxygruppe substituiertes Kohlenstoffatom oder die Carbonylgruppe darstellt:

Umlagerung eines N-Oxids der allgemeinen Formel

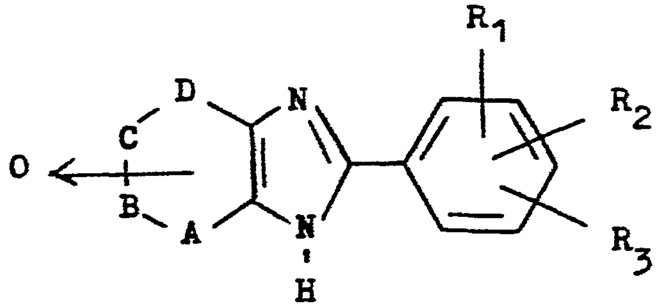

,(X)

in der
A bis D und $R_1$ bis $R_3$ wie eingangs definiert sind, und gegebenenfalls anschließende Hydrolyse.

Die Umsetzung wird gegebenenfalls in einem Lösungsmittel wie Benzol in Gegenwart eines Acylierungsmittels wie Essigsäureanhydrid oder Propionsäureanhydrid, wobei dieses zweckmäßigerweise gleichzeitig auch als Lösungsmittel verwendet wird, bei erhöhten Temperaturen, vorzugsweise jedoch bei der Siedetemperaur des verwendeten Lösungsmittels, durchgeführt wird.

Die gegebenenfalls anschließende Hydrolyse wird zweckmäßigerweise in Wasser, Wasser/Methanol, Wasser/Dioxan oder Methanol in Gegenwart einer Säure wie Salzsäure oder einer Base wie Natronlauge oder Ammoniak bei der Siedetemperatur des Reaktionsgemisches durchgeführt.

g) Zur Herstellung von Verbindungen der allgemeinen Formel I, in der mindestens einer der Reste A, B, C oder D ein durch eine Hydroxygruppe substituiertes Kohlenstoffatom oder die Carbonylgruppe und/oder mindestens einer der Reste $R_1$, $R_2$ oder $R_3$ eine Hydroxygruppe darstellen:

Entbenzylierung einer Verbindung der allgemeinen Formel

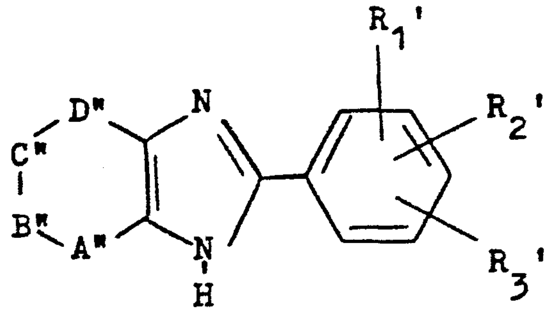

,(XI)

in der

einer der Reste A", B", C" oder D" ein durch eine Benzyloxygruppe substituiertes Kohlenstoffatom darstellt und die übrigen der Reste A" bis D" die für A bis D eingangs erwähnten Bedeutungen besitzen, und/oder mindestens einer der Reste $R_1$', $R_2$' oder $R_3$' eine Benzyloxygruppe darstellt und die übrigen der Reste $R_1$' bis $R_3$' die für $R_1$ bis $R_3$ eingangs erwähnten Bedeutungen besitzen.

Die Hydrogenolyse wird zweckmäßigerweise in einem Lösungsmittel wie Methanol, Ethanol, Isopropanol, Eisessig, Essigester, Dimethylformamid oder Wasser gegebenenfalls in Gegenwart einer Mineralsäure wie Salzsäure oder Bromwasserstoffsäure bei Temperaturen zwischen -10°C und 100°C, vorzugsweise bei 0°C bis 60°C in Gegenwart eines Katalysators wie Platin, Platinoxid- oder Palladium auf Kohle durchgeführt.

h) Zur Herstellung von Verbindungen der allgemeinen Formel I, in der mindestens einer der Reste $R_1$ bis $R_3$ eine Aminogrupe darstellt:

Hydrazinolyse einer Verbindung der allgemeinen Formel

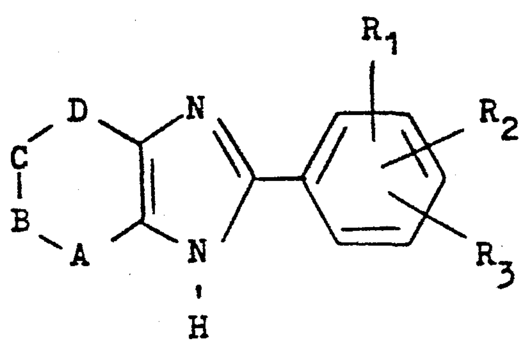

,(XII)

0149200

in der
A bis D wie eingangs definiert sind,
mindestens einer der Reste $R_1$, $R_2$ oder $R_3$ eine Gruppe der Formel

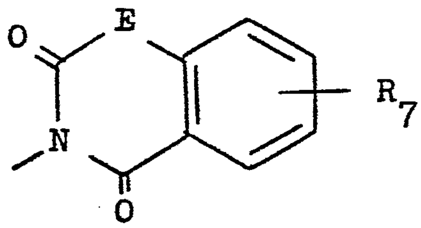

darstellt, wobei

E eine Bindung oder eine gegebenenfalls durch Alkylgruppen mit 1 bis 3 Kohlenstoffatomen substituierte Methylengruppe und
$R_7$ ein Wasserstoffatom oder eine Nitrogruppe bedeuten, und

der andere der Reste $R_1$ bis $R_3$ die für $R_1$ bis $R_3$ eingangs erwähnten Bedeutungen besitzt.

Die Hydrazinolyse wird zweckmäßigerweise mit Hydrazin oder Hydrazin-hydrat in einem Lösungsmittel wie Wasser, Methanol, Ethanol, Isopropanol, Tetrahydrofuran, Dioxan oder Ethylenglykoldimethylether oder aber auch ohne Lösungsmittel bei Temperaturen zwischen 0°C und der Siedetemperatur des Reaktionsgemisches durchgeführt.

Erhält man erfindungsgemäß eine Verbindung der allgemeinen Formel I, in der mindestens einer der Reste A, B, C oder D eine Alkoxymethingruppe und/oder mindestens einer der Reste $R_1$, $R_2$ oder $R_3$ eine Cyangruppe darstellt, so kann diese mittels Hydrolyse und/oder Alkoholyse in eine entsprechende Verbindung der allgemeinen Formel I, in der mindestens einer der Reste A, B, C oder D eine Carbonyl- oder

Hydroxymethingruppe und/oder mindestens einer der Reste $R_1$, $R_2$ oder $R_3$ eine Aminocarbonyl- oder Alkoxycarbonylgruppe darstellt, übergeführt werden und/oder

eine Verbindung der allgemeinen Formel I, in der mindestens einer der Reste $R_1$, $R_2$ oder $R_3$ eine Nitrogruppe darstellt, so kann diese mittels Reduktion in eine entsprechende Verbindung der allgemeinen Formel I, in der mindestens einer der Reste $R_1$, $R_2$ oder $R_3$ eine Aminogruppe darstellt, übergeführt werden und/oder

eine Verbindung der allgemeinen Formel I, in der mindestens einer der Reste $R_1$, $R_2$ oder $R_3$ eine Aminogruppe darstellt, so kann diese mittels Alkanoylierung in eine entsprechende Verbindung der allgemeinen Formel I, in der mindestens einer der Reste $R_1$, $R_2$ oder $R_3$ eine Alkanoylaminogruppe darstellt, übergeführt werden und/oder

eine Verbindung der allgemeinen Formel I, in der mindestens einer der Reste $R_1$, $R_2$ oder $R_3$ eine Aminogruppe darstellt, so kann diese über ihr Diazoniumsalz in eine entsprechende Verbindung der allgemeinen Formel I, in der einer der Reste $R_1$, $R_2$ oder $R_3$ ein Halogenatom, eine Hydroxy- oder Cyangruppe darstellt, übergeführt werden und/oder

eine Verbindung der allgemeinen Formel I, in der einer der Reste $R_1$, $R_2$ oder $R_3$ eine Aminocarbonylgruppe darstellt, so kann diese mittels Dehydratisierung in eine entsprechende Verbindung der allgemeinen Formel I, in der $R_1$ eine Cyangruppe darstellt.

Die nachträgliche Hydrolyse und/oder Alkoholyse wird in Gegenwart einer anorganischen Base mit Wasserstoffperoxid, z.B. mit 2 n Natronlauge oder Kalilauge/Wasserstoffperoxid,

in der Schmelze mit einer anorganischen Base, z.B. in einer Kaliumhydroxid-Schmelze, in Gegenwart einer Säure oder in Gegenwart einer alkoholischen Halogenwasserstoffsäure bei Temperaturen zwischen 0 und 50°C, vorzugsweise jedoch bei Raumtemperatur, durchgeführt.

Die nachträgliche Reduktion der Nitroverbindung wird vorzugsweise in einem Lösungsmittel wie Wasser, Wasser/Ethanol, Methanol, Eisessig, Essigsäureethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)-chlorid oder Natriumdithionit oder mit Hydrazin in Gegenwart von Raney-Nickel bei Temperaturen zwischen 0 und 50°C, vorzugsweise jedoch bei Raumtemperatur, durchgeführt.

Die nachträgliche Acylierung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid vorzugsweise mit einem reaktiven Derivat der Alkansäure, beispielsweise mit dem Säurechlorid oder dem Anhydrid, gegebenenfalls in Gegenwart einer anorganischen Base wie Natriumkarbonat oder einer tertiären organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels, durchgeführt.

Die nachträgliche Umsetzung eines Diazoniumsalzes, z.B. des Fluoroborats, des Fluorides in 40%iger Flußsäure, des Hydrosulfats in Schwefelsäure oder des Hydrochlorids, erforderlichenfalls in Gegenwart von Kupfer oder eines entsprechenden Kupfer-(I)-Salzes wie Kupfer-(I)-chlorid/Salzsäure oder Kupfer-(I)-bromid/Bromwasserstoffsäure, wird bei leicht erhöhten Temperaturen, z.B. bei Temperaturen zwischen 15°C und 100°C, durchgeführt. Das erforderliche Diazoniumsalz wird zweckmäßigerweise in einem geeigneten Lösungsmittel, z.B. in Wasser/Salzsäure, Methanol/Salzsäure, Äthanol/Salzsäure oder Dioxan/Salzsäure, durch Diazotierung einer entsprechenden Aminoverbindung mit einem Nitrit, z.B. Natriumnitrit oder einem Ester der salpetrigen Säure, bei niedrigen Temperaturen, z.B. bei Temperaturen zwischen -10°C und 5°C, hergestellt.

Die nachträgliche Dehydratisierung wird mit einem wasserentziehenden Mittel wie Phosphorpentoxid, Schwefelsäure oder p-Toluolsulfonsäurechlorid gegebenenfalls in einem Lösungsmittel wie Methylenchlorid oder Pyridin bei Temperaturen zwischen 0 und 100°C, vorzugsweise bei Temperaturen zwischen 20 und 80°C, durchgeführt.

Ferner können die erhaltenen Verbindungen der allgemeinen Formel I gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Furmarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis XII sind teilweise literaturbekannt bzw. erhält man nach literaturbekannten Verfahren.

So erhält man beispielsweise die als Ausgangsstoffe verwendeten Verbindungen der allgemeinen Formel II durch Acylierung einer entsprechenden o-Diaminoverbindung und die Verbindungen der allgemeinen Formeln III, IV, VI, VII und X bis XII durch anschließende Kondensation mit einem entsprechenden Benzoesäurederivat und gegebenenfalls anschließende Oxidation (siehe EP-A-0.022.495).

Wie bereits eingangs erwähnt weisen die neuen Verbindungen der allgemeinen Formel I, deren 1H Tautomere und deren physiologisch verträgliche Säureadditionssalze überlegene pharmakologische Eigenschaften insbesondere auch hinsichtlich der Wirkungsdauer auf, eine positiv inotrope Wirkung und/oder eine Wirkung auf den Blutdruck.

Beispielsweise wurden die Verbindungen

A = 2-(2-Methoxy-4-methylsulfonyl-phenyl)-4H-imidazo[4,5-b]-pyridin-5-on

B = 2-(2-Methoxy-4-methylmercapto-phenyl)-5H-imidazo[4,5-d]-pyridazin-4-on und

C = 8-(2-Methoxy-4-methylmercapto-phenyl)-purin-2-on

auf ihre biologischen Eigenschaften wie folgt untersucht:

Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparamter $dp/dt_{max}$ mittels eines Analog-

differenzierers gewonnen. Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 2 mg/kg i.v..

Die nachfolgende Tabelle enthält die Mittelwerte:

| Substanz | Dosis mg/kg i.v. | Zunahme von $dp/dt_{max}$ in % | Blutdruckwirkung in mm Hg | Wirkungsdauer (Halbwertszeit) in Minuten |
|---|---|---|---|---|
| A | 2,0 | + 85 | - 8/- 7 | 14 |
| B | 2,0 | + 72 | + 10/+ 10 | 22 |
| C | 0,6 | + 104 | + 58/+ 35 | 13 |

Die neuen Verbindungen sind gut verträglich, so konnte bei der Untersuchung der Substanzen A bis C keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und zum Teil durch die Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die

üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,3 - 2,2 mg/kg Körpergewicht, vorzugsweise jedoch 0,7 - 1,5 mg/kg Körpergewicht.

Die nachfolgenden Beispiele sollen die Erfindung näher erläutern:

Beispiel 1

2-(2-Methoxy-4-methylmercapto-phenyl)-5H-imidazo[4,5-d]pyridazin-4-on

---

2 g 2-Methoxy-4-methylmercapto-benzoesäure werden in 20 ml Polyphosphorsäure suspendiert. Dazu gibt man unter Rühren bei 50°C 1,2 g 4,5-Diamino-2H-pyridazin-3-on. Man erhitzt 90 Minuten auf 100-110°C, gießt dann auf Eiswasser und saugt das beim Verrühren ausfallende Produkt ab. Die Mutterlauge wird mit Essigester extrahiert, wonach man die wäßrige Phase ammoniakalisch stellt und wiederum mit Essigester extrahiert. Die letztere Essigesterphase wird eingedampft. Der Rückstand wird mit dem obigen Festprodukt vereinigt und durch Chromatographie auf Kieselgel (Elutionsmittel: Methylenchlorid/Ethanol = 100:0 bis 100:3) gereinigt.
Ausbeute: 0,34 g (12 % der Theorie),
Schmelzpunkt: 295-300°C (Zers.).

Beispiel 2

2-(2-Methoxy-4-methylsulfinyl-phenyl)-5H-imidazo[4,5-d]-pyridazin-4-on

---

0,21 g 2-(2-Methoxy-4-methylmercapto-phenyl)-5H-imidazo-[4,5-d]pyridazin-4-on werden in 10 ml Eisessig suspendiert und mit 0,08 ml 30%igem Wasserstoffperoxid veretzt. Die Mischung wird 24 Stunden bei Raumtemperatur gerührt und auf 20 ml Eiswasser gegossen. Man rührt dann solange bis sich ein Niederschlag gebildet hat. Dieser wird abgesaugt und durch Chromatographie an Kieselgel (Elutionsmittel: Methylenchlorid/Ethanol = 100:0 bis 100:3) gereinigt.
Ausbeute: 0,14 g (63 % der Theorie),
Schmelzpunkt: 285-288°C (Zers.).

Beispiel 3

2-(2-Methoxy-4-methylsulfonyl-phenyl)-5H-imidazo[4,5-d]pyridazin-4-on

0,07 g 2-(2-Methoxy-4-methylsulfinyl-phenyl)-5H-imidazo-[4,5-d]pyridazin-4-on werden in 5 ml Eisessig gelöst, mit 0,05 ml 30%igem Wasserstoffperoxid versetzt und zunächst 55 Minuten bei Raumtemperatur, dann 2 Stunden bei 40-50°C gerührt. Nun werden nochmals 0,05 ml Wasserstoffperoxid zugegeben, eine weitere Stunde erwärmt. Das ausgefallene Produkt wird abgesaugt und mit Ether gewaschen.
Ausbeute: 0,04 g (54 % der Theorie),
Schmelzpunkt: 308-312°C (Zers.).

Beispiel 4

2-(2-Dimethylamino-4-nitro-phenyl)-5-methoxy-imidazo[4,5-b]-pyridin

a) 3-[(2-Dimethylamino-4-nitro-benzoyl)-amino]-2-amino-6-methoxy-pyridin

1,05 g 2-Dimethylamino-4-nitro-benzoesäure werden in 35 ml Phosphoroxychlorid suspendiert und dazu 1,05 g 2,3-Diamino-6-methoxy-pyridin-dihydrochlorid gegeben. Man erhitzt für 5 Minuten zum Rückfluß, läßt 15 Minuten unter Rühren abkühlen und zersetzt schließlich mit Wasser. Die entstandene Lösung wird abgekühlt und auf eine Mischung aus Eis und konzentriertem Ammoniak gegossen. Das Produkt fällt hierbei aus. Es wird abgesaugt, mit Wasser gewaschen und im Umlufttrockenschrank bei 50°C getrocknet.
Ausbeute: 1,5 g (92 % der Theorie),
Schmelzpunkt: 218-220°C (Zers.).

b) 2-(2-Dimethylamino-4-nitro-phenyl)-5-methoxy-imidazo-[4,5-b]pyridin

Eine Suspension von 1,4 g 3-[(2-Dimethylamino-4-nitro-benzoyl)-amino]-2-amino-6-methoxy-pyridin in 40 ml Phosphoroxychlorid wird 3 Stunden zum Rückfluß erhitzt. Das Phosphoroxychlorid wird zu etwa 2/3 abgedampft und der Rückstand auf Wasser gegossen. Die entstandene Lösung wird ammoniakalisch gestellt und das ausgefallene Produkt über eine Kieselgelsäule gereinigt (Elutionsmittel: Methylenchlorid/Ethanol = 100:0 bis 100:0,5).
Ausbeute: 0,85 g (64 % der Theorie),
Schmelzpunkt: 183-186°C.

Beispiel 5

2-(2-Dimethylamino-4-nitro-phenyl)-5-methoxy-imidazo[4,5-b]-pyridin

0,45 g 2-Dimethylamino-4-nitro-benzoesäure werden in 15 ml Phosphoroxychlorid suspendiert und dazu 0,45 g 2,3-Diamino-6-methoxy-pyridin-dihydrochlorid gegeben. Die Mischung wird 2 Stunden zum Rückfluß erhitzt und nach dem Abkühlen auf Wasser gegossen. Die wäßrige Lösung wird unter Kühlung ammoniakalisch gestellt und das ausgefallene Produkt wie in Beispiel 4b behandelt.
Ausbeute: 0,3 g (46 % der Theorie),
Schmelzpunkt: 183-186°C.

Beispiel 6

2-(2-Dimethylamino-4-nitro-phenyl)-4H-imidazo[4,5-b]pyridin-5-on

0,2 g 2-(2-Dimethylamino-4-nitro-phenyl)-5-methoxy-imidazo-[4,5-b]pyridin werden im Bombenrohr 3 Stunden mit 5 ml konzentrierter Salzsäure auf 100°C erhitzt. Das Reaktionsgemisch wird auf etwa 20 ml Eis gegossen, neutralisiert und mit Essigester extrahiert. Nach Waschen mit Wasser und Trocknen mit Magnesiumsulfat wird die Essigesterphase eingedampft. Der Rückstand wird durch Verreiben in Ether/Petrolether kristallisiert.
Ausbeute: 0,06 g (31 % der Theorie),
Schmelzpunkt: 313-316°C (Zers.).

Beispiel 7

2-(2-Methoxy-4-methylmercapto-phenyl)-4H-imidazo[4,5-b]-pyridin-5-on

6,2 g 2-[(2-Methoxy-4-methylmercapto-benzoyl)-amino]-3-nitro-1H-pyridin-6-on werden in 370 ml Eisessig gelöst und mit Wasserstoff in Gegenwart von 6 g 10%iger Palladiumkohle bei Raumtemperatur und 5 bar Druck hydriert (Reaktionsdauer: 2 Stunden). Der Katalysator wird abfiltriert und das Filtrat 1,5 Stunden unter Rühren zum Rückfluß erhitzt. Anschließend werden etwa 250 ml des Lösungsmittels abgedampft, die verbleibende Lösung auf Eiswasser gegossen und mit Ammoniak neutralisiert. Der ausgefallene Niederschlag wird aus Methanol umkristallisiert. Eine weitere Fraktion kann durch Eindampfen der Kristallisationsmutterlaugen und Verreiben des Rückstandes mit Ether gewonnen werden.
Ausbeute: 3,4 g (60 % der Theorie),
Schmelzpunkt: 274-275°C.

Beispiel 8

2-(2-Methoxy-4-methylsulfinyl-phenyl)-4H-imidazo[4,5-b]pyridin-5-on

Hergestellt analog Beispiel 2 durch Oxidation von 2-(2-Methoxy-4-methylmercapto-phenyl)-4H-imidazo[4,5-b]pyridin-5-on. Schmelzpunkt: 250-255°C (Zers.).

Beispiel 9

2-(2-Methoxy-4-methylsulfonyl-phenyl)-4H-imidazo[4,5-b]pyridin-5-on

Hergestellt analog Beispiel 3 durch Oxidation von 2-(2-Methoxy-4-methylsulfinyl-phenyl)-4H-imidazo[4,5-b]pyridin-5-on. Schmelzpunkt: 300°C (Zers.).

Beispiel 10

2-(2-Methoxy-4-dimethylaminosulfonyl-phenyl)-4H-imidazo-[4,5-b]-pyridin-5-on

Hergestellt analog Beispiel 7 aus 2-(2-Methoxy-4-dimethylaminosulfonyl-benzoyl-amino)-3-nitro-1H-pyridin-6-on.
Schmelzpunkt: 283-285°C.

Beispiel 11

2-(2,4-Dimethoxy-5-aminosulfonyl-phenyl)-4H-imidazo[4,5-b]-pyridin-5-on

1,36 g 2-(2,4-Dimethoxy-phenyl)-4H-imidazo[4,5-b]pyridin-5-on werden bei 0°C in 5 ml Chlorsulfonsäure eingetragen und die Mischung 24 Stunden bei 20-30°C gerührt. Man gießt auf Eis und filtriert den entstandenen Niederschlag ab. Er wird portionsweise in 100 ml konzentriertem Ammoniak eingetragen und über Nacht bei Raumtemperatur gerührt. Man filtriert vom verbleibenden Niederschlag ab und stellt das Filtrat mit Eisessig auf pH 5-6. Der entstandene Niederschlag wird abzentrifugiert und mit Wasser digeriert. Der hierbei verbleibende Niederschlag wird abfiltriert und mit Aceton verrührt.
Ausbeute: 0,04 g (2 % der Theorie),
Schmelzpunkt: 280-285°C (Zers.).

Beispiel 12

2-(2,4-Dimethoxy-5-hydroxsulfonyl-phenyl)-4H-imidazo[4,5-b]-pyridin-5-on

Erhalten durch Eindampfen der beim Digerieren erhaltenen wäßrigen Phase gemäß Beispiel 11 und Umkristallisation aus 2n Salzsäure. Das Produkt fällt hierbei als Halbhydrat an.
Ausbeute: 0,11 g (6 % der Theorie),
Schmelzpunkt: 295-300°C (Zers.).

## Beispiel 13

### 2-(2,4-Dimethoxy-phenyl)-4H-imidazo[4,5-b]pyridin-5-on

4 g 2-(2,4-Dimethoxy-phenyl)-imidazo[4,5-b]pyridin-4-oxid werden in 40 ml Acetanhydrid suspendiert und 2,75 Stunden zum Rückfluß erhitzt. Nach dem Abkühlen wird auf 150 ml Eis gegossen, 25 ml 6 n Salzsäure zugegeben und das Gemisch 0,5 Stunden bei 120°C gerührt. Es wird auf Raumtemperatur abgekühlt, 50 ml Wasser zugefügt und der Niederschlag abgesaugt. Das Produkt wird durch Chromatographie auf Kieselgel (Elutionsmittel: Methylenchlorid/Methanol = 100:0 bis 100:10) gereinigt.

Ausbeute: 3 g (75 % der Theorie),

Schmelzpunkt: 270-275°C (Zers.).

## Beispiel 14

### 8-(2-Methoxy-4-methylmercapto-phenyl)-1,3-dimethyl-1H,3H-purin-2,6-dion

8,51 g 4,5-Diamino-1,3-dimethyl-uracil, 9,91 g 2-Methoxy-4-methylmercapto-benzoesäure werden zusammen mit 200 ml Phosphoroxychlorid 5,5 Stunden zum Rückfluß erhitzt, überschüssiges Phosphoroxychlorid wird dann abgedampft und der Rückstand mit Eiswasser zersetzt. Der ausgefallene Niederschlag wird aus Ethanol umkristallisiert.

Ausbeute: 14,7 g (89 % der Theorie),

Schmelzpunkt: über 280°C

| | | | | |
|---|---|---|---|---|
| Ber.: | C 54,20 | H 4,85 | N 16,86 | S 9,65 |
| Gef.: | 54,24 | 4,60 | 16,67 | 9,89 |

Beispiel 15

8-(2-Methoxy-4-methylsulfinyl-phenyl)-1,3-dimethyl-1H,3H-purin-2,6-dion

3,7 g 8-(2-Methoxy-4-methylmercapto-phenyl)-1,3-dimethyl-1H,3H-purin-2,6-dion werden in 30 ml 50%iger Essigsäure suspendiert und dazu 1,8 g wasserfreies Natriumacetat gegeben. Das Gemisch wird unter Rühren tropfenweise mit 1,6 g Brom (gelöst in 1,5 ml Eisessig) versetzt. Man rührt 20 Minuten nach, gießt auf Eis, stellt ammoniakalisch und kristallisiert den Niederschlag aus Ethanol um.
Ausbeute: 2,1 g (55 % der Theorie),
Schmelzpunkt: 228-229°C.

Beispiel 16

8-(2,4-Dimethoxy-phenyl)-1H-purin-6-on

0,5 g 8-(2,4-Dimethoxy-phenyl)-6-chlor-purin werden in 30 ml 2n Natronlauge 7 Stunden zum Rückfluß erhitzt. Man säuert mit Eisessig an und kristallisiert den ausgefallenen Niederschlag aus Methanol um.
Ausbeute: 0,11 g (23 % der Theorie),
Schmelzpunkt: über 270°C

| | | | | | | |
|---|---|---|---|---|---|---|
| Ber.: | C | 57,34 | H | 4,44 | N | 20,58 |
| Gef.: | | 57,60 | | 4,48 | | 20,58 |

Beispiel 17

8-(5-Aminosulfonyl-2,4-dimethoxy-phenyl)-purin-6-on

Hergestellt analog Beispiel 16 aus 6-Chlor-8-(5-amino-sulfonyl-2,4-dimethoxy-phenyl)-purin durch Umsetzung mit 20%iger Kalilauge.
Ausbeute: 42 % der Theorie,
Schmelzpunkt: 262-265°C.

Beispiel 18

8-(2-Methoxy-5-methylmercapto-phenyl)-purin-6-on

Hergestellt analog Beispiel 16 aus 6-Chlor-8-(2-methoxy-5-methylmercapto-phenyl)-purin durch Umsetzung mit 20%iger Kalilauge.
Ausbeute: 53 % der Theorie,
Schmelzpunkt: 187-190°C.

Beispiel 19

8-(5-Brom-2,4-dimethoxy-phenyl)-purin-6-on

Hergestellt analog Beispiel 16 aus 6-Chlor-8-(5-brom-2,4-dimethoxy-phenyl)-purin durch Umsetzung mit 20%iger Kalilauge.
Ausbeute: 78,9 % der Theorie,
Schmelzpunkt: 244-246°C.

Beispiel 20

8-(4-Chlor-2-methoxy-5-methylaminosulfonyl-phenyl)-purin-6-on

Hergestellt analog Beispiel 16 aus 6-Chlor-(4-chlor-2-methoxy-5-methylamino-sulfonyl-phenyl)-purin durch Umsetzung mit 20%iger Kalilauge.
Ausbeute: 47 % der Theorie,
Schmelzpunkt: 213-216°C.

Beispiel 21

8-(2-Methoxy-5-methylsulfinyl-phenyl)-purin-6-on

Hergestellt analog Beispiel 2 durch Oxidation von 8-(2-Methoxy-5-methylmercapto-phenyl)-purin-6-on.
Ausbeute: 66 % der Theorie,
Schmelzpunkt: 203-206°C.

Beispiel 22

8-(2-Methoxy-4-methylsulfinyl-phenyl)-purin-2,6-dion

Hergestellt analog Beispiel 2 durch Oxidation von 8-(2-Methoxy-4-methylmercapto-phenyl)-purin-2,6-dion.
Ausbeute: 49 % der Theorie,
Schmelzpunkt: > 315°C.

| | | | | | | | | |
|---|---|---|---|---|---|---|---|---|
| Ber.: | C | 48,74 | H | 3,77 | N | 17,49 | S | 10,01 |
| Gef.: | | 48,27 | | 3,72 | | 17,35 | | 9,74 |

Beispiel 23

8-(2-Methoxy-4-methylsulfinyl-phenyl)-purin-2-on-hydrochlorid

Hergestellt analog Beispiel 2 durch Oxidation von 8-(2-Methoxy-4-methylmercapto-phenyl)-purin-2-on-hydrochlorid.
Ausbeute: 20 % der Theorie,
Schmelzpunkt: 265-268°C (Zers.).

Beispiel 24

8-(2-Methoxy-5-methylsulfonyl-phenyl)-purin-6-on

Hergestellt analog Beispiel 3 durch Oxidation von 8-(2-Methoxy-5-methylmercapto-phenyl)-purin-6-on mit Wasserstoffperoxid in Ameisensäure.
Ausbeute: 65 % der Theorie,
Schmelzpunkt: 267-269°C.

Beispiel 25

8-(2-Methoxy-4-methylsulfonyl-phenyl)-purin-2,6-dion

Hergestellt analog Beispiel 3 durch Oxidation von 8-(2-Methoxy-4-methylmercapto)-phenyl-purin-2,6-dion mit Wasserstoffperoxid in Ameisensäure.
Ausbeute: 20 % der Theorie,
Schmelzpunkt: > 315°C.

| | | | | | | | | |
|---|---|---|---|---|---|---|---|---|
| Ber.: | C | 46,43 | H | 3,59 | N | 16,66 | S | 9,53 |
| Gef.: | | 43,95 | | 3,93 | | 16,06 | | 9,84 |

Beispiel 26

8-(2-Methoxy-4-methylmercapto-phenyl)-purin-2-on

Hergestellt analog Beispiel 6 aus 2-Methoxy-8-(2-methoxy-4-methylmercapto-phenyl)-purin.
Ausbeute: 81 % der Theorie,
Schmelzpunkt: > 300°C.

| | | | | | | | | |
|---|---|---|---|---|---|---|---|---|
| Ber.: | C | 54,15 | H | 4,19 | N | 19,43 | S | 11,12 |
| Gef.: | | 53,93 | | 4,52 | | 19,39 | | 11,11 |

Beispiel 27

8-(2-Methoxy-4-hydroxy-phenyl)-purin-2-on

Hergestellt analog Beispiel 7 aus 4-(4-Benzyloxy-2-methoxy-benzoyl-amino)-5-nitro-pyrimidin-2-on.
Ausbeute: 72 % der Theorie,
Schmelzpunkt: > 330°C.

| | | | | | | |
|---|---|---|---|---|---|---|
| Ber.: | C | 52,17 | H | 4,39 | N | 21,70 |
| Gef.: | | 52,20 | | 4,26 | | 22,02 |

Beispiel 28

8-(2-Methoxy-4-methylmercapto-phenyl)-purin-2,6-dion

a) 4-Amino-5-(2-methoxy-4-methylmercapto-benzoylamino)-pyrimidin-2,6-dion

20 g 2-Methoxy-4-methylmercapto-benzoesäure werden in 260 ml Dimethylformamid gelöst, mit 13 g N,N'-Carbonyl-diimidazol versetzt und 30 Minuten bei Raumtemperatur gerührt. Es werden 13 g 4,5-Diamino-pyrimidin-2,6-dion zugefügt und das Reaktionsgemisch 3 Stunden unter Rühren am Rückfluß gekocht. Nach dem Abkühlen wird in 1 l Wasser gegossen. Das ausgefallene Produkt wird abgesaugt, mit Wasser gewaschen und im Umlufttrockenschrank bei 70°C getrocknet.
Ausbeute: 17,7 g (60 % der Theorie),
Schmelzpunkt: > 320°C.

b) 8-(2-Methoxy-4-methylmercapto-phenyl)-purin-2,6-dion

2 g 4-Amino-5-(2-methoxy-4-methylmercapto-benzoylamino)-pyrimidin-2,6-dion werden mit 50 ml Ethanol und 50 ml 2 n Natronlauge 21 Stunden am Rückfluß gekocht. Hierbei tritt nach ca. 15 Minuten Auflösung ein. Nach dem Abkühlen wird mit Eisessig abgesaugt, mit Waser gewaschen und im Umlufttrockenschrank bei 70°C getrocknet. Das so erhaltene Rohprodukt wird durch Chromatographie auf Kieselgel (Elutionsmittel: Methylenchlorid/Ethanol = 19:1, 9:1, 4:1 und Ethanol) gereinigt.
Ausbeute: 0,95 g (50 % der Theorie),
Schmelzpunkt: 312-315°C.

Beispiel 29

8-(2-Methoxy-4-dimethylamino-sulfonyl-phenyl)-purin-2,6-dion

Hergestellt analog Beispiel 28b aus 4-Amino-5-(2-methoxy-4-dimethylaminosulfonyl-benzoylamino)-pyrimidin-2,6-dion.
Ausbeute: 34 % der Theorie,
Schmelzpunkt: 323-325°C.

Beispiel 30

8-(5-Aminosulfonyl-2,4-dimethoxy-phenyl)-2-methoxy-purin

Hergestellt analog Beispiel 11 aus 2-Methoxy-8-(2,4-dimethoxy-phenyl)-purin.
Ausbeute: 45 % der Theorie,
Schmelzpunkt: 267-270°C

Beispiel 31

8-(5-Aminosulfonyl-2,4-dimethoxy-phenyl)-purin-2-on

Hergestellt analog Beispiel 6 aus 2-Methoxy-8-(2,4-dimethoxy-5-aminosulfonyl-phenyl)-purin.
Ausbeute: 35 % der Theorie (als Hydrochlorid),
Schmelzpunkt: 270-272°C (Zers.).

Beispiel 32

8-(2-Dimethylamino-4-nitro-phenyl)-purin-6-on

1 g 5-Amino-4-(2-dimethylamio-4-nitro-benzoylamino)-pyrimidin-6-on werden in 50 ml Eisessig 2 Stunden am Rückfluß erhitzt. Es wird zur Trockene eingedampft und der Rückstand mit Ethanol in der Wärme ausgerührt.
Ausbeute: 0,52 g (54 % der Theorie),
Schmelzpunkt: > 300°C

| | | | | | | |
|---|---|---|---|---|---|---|
| Ber.: | | 51,98 | | 4,03 | | 27,98 |
| Gef.: | C | 51,67 | H | 4,10 | N | 27,76 |

Beispiel 33

8-(4-Amino-2-dimethylamino-phenyl)-purin-6-on

0,9 g 8-(2-Dimethylamino-4-nitro-phenyl)-purin-6-on werden in 50 ml Methanol in Gegenwart von 0,5 g Raney-Nickel bei Raumtemperatur und 5 bar mit Wasserstoff 3 Stunden lang bis zur Aufnahme der berechneten Menge hydriert. Nach dem Absaugen des Katalysators wird das Filtrat im Vakuum zur Trockene eingedampft.
Ausbeute: 0,74 g (91 % der Theorie),
Schmelzpunkt: 232-235°C.

Beispiel 34

8-(4-Acetamido-2-dimethylamino-phenyl)-purin-6-on

0,6 g 8-(4-Amino-2-dimethylamino-phenyl)-purin-6-on werden in 5 ml Acetanhydrid 1 Stunde auf 150°C erhitzt. Man dampft im Vakuum zur Trockene ein und kristallisiert aus Aceton/-Ether um.
Ausbeute: 0,17 g (25 % der Theorie),
Schmelzpunkt: 278-280°C.

Beispiel 35

8-(2-Methoxy-4-methylmercapto-phenyl)-purin-6-on

Hergestellt analog Beispiel 28b aus 4-Amino-5-(2-methoxy-4-methylmercapto-benzoylamino)-pyrimidin-6-on.
Ausbeute: 5 % der Theorie,
Schmelzpunkt: 293-295°C.

Beispiel 36

8-(4-Benzyloxy-2-methoxy-phenyl)-purin-6-on

Hergestellt analog Beispiel 16 aus 8-(4-Benzyloxy-2-methoxy-phenyl)-6-chlor-purin durch Umsetzung mit 40%iger Kalilauge.
Ausbeute: 52 % der Theorie (als amorphe Substanz),
Schmelzpunkt: ab 150°C

| | | | | | | |
|---|---|---|---|---|---|---|
| Ber.: | C | 65,51 | H | 4,63 | N | 16,08 |
| Gef.: | | 65,28 | | 4,64 | | 16,13 |

Beispiel 37

2-(2-Dimethylamino-4-amino-phenyl)-4H-imidazo[4,5-b]pyridin-5-on-hydrochlorid

1,7 g 2-(2-Dimethylamino-4-nitro-phenyl)-4H-imidazo[4,5-b]-pyridin-5-on werden in 20 ml Dimethylformamid gelöst und mit 0,2 g 10%iger Palladiumkohle versetzt. Man hydriert 3 Stunden mit Wasserstoff bei Raumtemperatur und 5 bar Druck. Man filtriert vom Katalysator ab, versetzt die Mutterlauge mit je 100 ml Ether und Essigester und fügt schließlich 3 ml etherische Salzsäure hinzu. Der Niederschlag wird aus Methanol umkristallisiert. Nach Trocknung über Phosphorpentoxid und Kaliumhydroxid erhält man ein Produkt, das auf ein Mol der Base 1,5 Mol Salzsäure und ein halbes Mol Wasser enthält.
Ausbeute: 0,42 g (25 % der Theorie),
Schmelzpunkt: 225-227°C (Zers.).

Beispiel 38

2-(2-Dimethylamino-4-acetamino-phenyl)-4H-imidazo[4,5-b]-pyridin-5-on

0,6 g 2-(2-Dimethylamino-4-amino-phenyl)-imidazo-[4,5-b]-pyridin-5-on werden mit 20 ml Acetanhydrid 30 Minuten zum Rückfluß erhitzt. Nach Eindampfen versetzt man mit 10 ml konzentriertem Ammoniak und schließlich mit 20 ml Eis. Das

ausgefallene Produkt wird abgesaugt und mit Wasser gewaschen.
Ausbeute: 0,4 g (58 % der Theorie),
Schmelzpunkt: 309-320°C (Zers.).

Beispiel 39

8-(2-Methoxy-4-benzyloxy-phenyl)-2-benzyloxy-purin

a) 8-(2-Methoxy-4-benzyloxy-phenyl)-2-chlor-purin

Hergestellt analog Beispiel 5 aus 2-Chlor-4,5-diamino-pyrimidin und 2-Methoxy-4-benzyloxy-benzoesäure.
Schmelzpunkt: 245-246°C.

b) 8-(2-Methoxy-4-benzyloxy-phenyl)-2-benzyloxy-purin

4,5 g gepulvertes Kaliumhydroxid werden in 70 ml Benzylalkohol gelöst und anschließend 7,2 g 8-(2-Methoxy-4-benzyloxy-phenyl)-2-chlor-purin zugegeben. Man erhitzt 3 Stunden auf 150°C, kühlt ab, filtriert, versetzt das Filtrat mit Ether und reinigt den ausgefallenen Niederschlag durch Chromatographie an Kieselgel (Elutionsmittel: Methylenchlorid/Ethanol = 100:2,5).
Ausbeute: 5,58 g (65 % der Theorie)
Schmelzpunkt: 152-153°C.

Beispiel 40

8-(2-Methoxy-4-hydroxy-phenyl)-3H-purin-2-on

3 g 8-(2-Methoxy-4-benzyloxy-phenyl)-2-benzyloxy-purin werden in 100 ml Ethanol gelöst und in Gegenwart von 1 g 20%iger Palladiumkohle 2 Stunden bei 50°C mit Wasserstoff von 5 bar hydriert. Der Katalysator wird abfiltriert und mit

heißem Ethanol gewaschen. Die Filtrate werden eingedampft und der Rückstand mit Methylenchlorid verrieben. Das erhaltene Festprodukt wird mit 30 ml 2 n Natronlauge verrührt. Die Lösung wird filtriert und das Filtrat mit Eisessig angesäuert. Der gebildete Niederschlag wird durch Zentrifugieren von der Lösung abgetrennt und durch Verreiben mit Aceton gereinigt. Das Produkt enthält 1 Mol Kristallwaser.
Ausbeute: 0,22 g (12 % der Theorie),
Schmelzpunkt: Zersetzung ab 250°C.

Beispiel 41

2-(2-Methoxy-4-amino-5-nitro-phenyl)-5-methoxy-imidazo-[4,5-b]pyridin

0,5 g 2-[2-Methoxy-5-nitro-4-(4,4-dimethyl-7-nitro-2H,4H-isochinolin-1,3-dion-2-yl)-phenyl]-5-methoxy-imidazo[4,5-b]-pyridin werden in 10 ml Isopropanol suspendiert, mit 0,5 ml 80%igem Hydrazinhydrat versetzt und unter Rühren 1,75 Stunden zum Rückfluß erhitzt. Das Reaktionsgemisch wird eingedampft, der Rückstand mit 50 ml Eiswasser verrieben und das ausgefallene Produkt durch Chromatographie an Kieselgel (Elutionsmittel: Methylenchlorid/Ethanol = 100:0 bis 100:2) gereinigt.
Ausbeute: 0,18 g (57 % der Theorie),
Schmelzpunkt: 250-251°C (Zers.).

Beispiel 42

2-(2-Methoxy-4-amino-5-nitro-phenyl)-4H-imidazo[4,5-b]pyridin-5-on-hydrochlorid

Hergestellt analog Beispiel 6 aus 2-(2-Methoxy-4-amino-5-nitro-phenyl)-5-methoxy-imidazo[4,5-b]pyridin. Das Produkt fällt aus dem Reaktionsgemisch aus.
Schmelzpunkt: Zersetzung ab 270°C.

Beispiel 43

2-(2-Methoxy-4-methylmercapto-phenyl)-7H-imidazo[4,5-e]-1,2,4-triazin-6-on

---

1,27 g 5,6-Diamino-2H-1,2,4-triazin-3-on werden in 50 ml Pyridin 0,5 Stunden unter Rühren zum Rückfluß erhitzt. Anschließend werden 4,33 g 2-Methoxy-4-methylmercapto-benzoylchlorid zugegeben und weitere 2 Stunden zum Rückfluß erhitzt. Man saugt den Niederschlag heiß ab und wäscht mit Wasser nach. Das erhaltene Festprodukt wird in 2n Natronlauge 15 Minuten bei Raumtemperatur gerührt. Das verbleibende Festprodukt wird abfiltriert, das Filtrat mit Eisessig neutralisiert und der ausgefallene Niederschlag mit Wasser ausgekocht.
Ausbeute: 0,1 g (3 % der Theorie),
Schmelzpunkt: Zersetzung ab 317°C.

Beispiel 44

2-(2,4-Dimethoxy-phenyl)-7H-imidazo[4,5-e]-1,2,4-triazin-6-on

---

Hergestellt analog Beispiel 43 aus 5,6-Diamino-2H-1,2,4-triazin-3-on und 2,4-Dimethoxy-benzoylchlorid.
Schmelzpunkt: Zersetzung ab 305°C.

Beispiel 45

2-(2-Methoxy-4-amino-phenyl)-5H-imidazo[4,5-d]pyridazin-4-on

Hergestellt analog Beispiel 1 aus 4,5-Diamino-2H-pyridazin-3-on und 2-Methoxy-4-amino-benzoesäure.
Schmelzpunkt: 295-300°C (Zers.).

Beispiel 46

8-(2-Methoxy-5-methylmercapto-phenyl)-3H-purin-2-on

Hergestellt analog Beispiel 32 aus 4-Amino-5-(2-methoxy-5-methylmercapto-benzoylamino)-1H-pyrimidin-2-on.
Schmelzpunkt: über 330°C,

| | | | | | | | | |
|---|---|---|---|---|---|---|---|---|
| Ber.: | C | 54,15 | H | 4,20 | N | 19,43 | S | 11,12 |
| Gef.: | | 54,30 | | 4,40 | | 19,07 | | 11,28 |

Beispiel 47

8-(2,4-Dimethoxy-5-brom-phenyl)-3H-purin-2-on

Hergestellt analog Beispiel 32 aus 4-Amino-5-(2,4-dimethoxy-5-brom-benzoylamino)-1H-pyrimidin-2-on.
Schmelzpunkt: 292-294°C (Zers.).

Beispiel 48

8-(5-Aminosulfonyl-2,4-dimethoxy-phenyl)-1H,3H-purin-2,6-dion

Hergestellt analog Beispiel 28b aus 4-Amino-5-(5-aminosulfonyl-2,4-dimeth-oxy-benzoylamino)-1H,3H-pyrimidin-2,6-dion.
Schmelzpunkt: 325-328°C.

Beispiel 49

8-(4-Aminosulfonyl-2-methoxy-phenyl)-1H,3H-purin-2,6-dion

Hergestellt analog Beispiel 28b aus 4-Amino-5-(4-amino-sulfonyl-2-methoxy-benzoylamino)-1H,3H-pyrimidin-2,6-dion.
Schmelzpunkt: > 330°C.

| | | | | | | | | |
|---|---|---|---|---|---|---|---|---|
| Ber.: | C | 42,73 | H | 3,29 | N | 21,76 | S | 9,50 |
| Gef.: | | 42,44 | | 3,50 | | 21,6 | | 9,69 |

Beispiel 50

8-(2-Methoxy-4-methylaminosulfonyl-phenyl)-1H,3H-purin-2,6-dion

Hergestellt analog Beispiel 28b aus 4-Amino-5-(2-methoxy-4-methylaminosulfonyl-benzoylamino)-1H,3H-pyrimidin-2,6-dion.
Schmelzpunkt: 238-240°C (Zers.).

Beispiel 51

8-(4-Carboxy-2-methoxy-phenyl)-1H,3H-purin-2,6-dion

Hergestellt analog Beispiel 28b aus 4-Amino-5-(4-carboxy-2-methoxybenzoylamino)-1H,3H-pyrimidin-2,6-dion.
Schmelzpunkt: > 330°C.

| | | | | | | |
|---|---|---|---|---|---|---|
| Ber.: | C | 49,66 | H | 3,47 | N | 19,30 |
| Gef.: | | 49,65 | | 3,56 | | 19,49 |

Beispiel 52

2-(2-Propoxy-4-methylmercapto-phenyl)-5-methoxy-imidazo-[4,5-b]pyridin-hydrochlorid

Hergestellt analog Beispiel 5 aus 2,3-Diamino-6-methoxy-pyridin-dihydrochlorid und 2-Propoxy-4-methylmercapto-benzoesäure.
Schmelzpunkt: Zersetzung ab 178°C.

Beispiel 53

2-(2-Propoxy-4-methylmercapto-phenyl)-4H-imidazo[4,5-b]pyridin-5-on-hydrochlorid

Hergestellt analog Beispiel 6 aus 2-(2-Propoxy-4-methylmercapto-phenyl)-5-methoxy-imidazo[4,5-b]pyridin-hydrochlorid.
Schmelzpunkt: 245-247°C (Zers.).

Beispiel 54

2-(2-Methoxy-4-chlor-phenyl)-5H-imidazo[4,5-b]pyridazin-4-on

1,05 g 2-(2-Methoxy-4-amino-phenyl)-5H-imidazo[4,5-d]pyridazin-4-on werden in 20 ml 6n Salzsäure suspendiert. Die Suspension wird auf 0 bis -3°C abgekühlt und 0,28 g Natriumnitrit, gelöst in 2 ml Wasser, zugetropft. Man rührt nach 40 Minuten nach und erhitzt dann eine Stunde auf 80-85°C. Das beim Abkühlen ausfallende Produkt wird durch Chromatographie auf Kieselgel (Elutionsmittel: zunächst Methylenchlorid/-

Ethanol = 100:0 bis 100:20 dann Zusatz von 1,5 bis 2,5 % Eisessig).
Ausbeute: 0,11 g (10 % der Theorie),
Schmelzpunkt: 300-303°C (Zers.).

Beispiel 55

2-(2-Methoxy-4-hydroxy-phenyl)-5H-imidazo[4,5-d]pyridazin-4-on

Die Verbindung wird aus dem in Beispiel 54 beschriebenen Ansatz ab zweite Fraktion bei der Säulentrennung erhalten.
Ausbeute: 0,6 g (57 % der Theorie),
Schmelzpunkt: 310-315°C (Zers.).

Beispiel 56

2-(2-Propoxy-4-methylsulfinyl-phenyl)-4H-imidazo[4,5-b]pyridin-5-on-hydrochlorid

Hergestellt analog Beispiel 2 aus 2-(2-Propoxy-4-methylmercapto-phenyl)-4H-imidazo[4,5-b]pyridin-5-on.
Schmelzpunkt: ab 188°C (Zers.).

Beispiel 57

2-(2-Propoxy-4-methylsulfonyl-phenyl)-4H-imidazo[4,5-b]pyridin-5-on-hydrochlorid

Hergestellt analog Beispiel 3 aus 2-(2-Propoxy-4-methylmercapto-phenyl)-4H-imidazo[4,5-b]pyri-din-5-on-hydrochlorid.
Schmelzpunkt: 312-314°C.

Beispiel 58

2-(2-Methoxy-4-dimethylaminosulfonyl-phenyl)-5H-imidazo-[4,5-c]pyridin-4-on

Hergestellt durch 2-stündiges Erhitzen von 1,1 g 5-Amino-4-(2-methoxy-4-dimethylaminosulfonyl-benzoylamino)-1H-pyridin-2-on in 5 g Polyphosphorsäure.
Schmelzpunkt: 192-194°C.

Beispiel 59

8-(2-Methoxy-4-aminosulfonyl-phenyl)-3H-purin-2-on

Hergestellt analog Beispiel 32 aus 4-Amino-5-(2-methoxy-4-aminosulfonyl-benzoylamino)-3H-pyrimidin-2-on.
Schmelzpunkt: 255-257°C.

Beispiel 60

8-(2-Methoxy-4-methylaminosulfonyl-phenyl)-3H-purin-2-on

Hergestellt analog Beispiel 32 aus 4-Amino-5-(2-methoxy-4-methylaminosulfonyl-benzoylamino)-3H-pyrimidin-2-on.
Schmelzpunkt: 264-266°C.

Beispiel 61

8-(2-Methoxy-4-dimethylaminosulfonyl-phenyl)-3H-purin-2-on

Hergestellt analog Beispiel 32 aus 4-Amino-5-(2-methoxy-4-dimethylaminosulfonyl-benzoylamino)-3H-pyrimidin-2-on. Schmelzpunkt: 296-298°C.

Beispiel 62

8-(2-Methoxy-4-cyan-phenyl)-3H-purin-2-on

Hergestellt analog Beispiel 32 aus 4-Amino-5-(2-methoxy-4-cyan-benzoylamino)-3H-pyrimidin-2-on.
Schmelzpunkt: 307-309°C.

Beispiel 63

8-(2-Methoxy-4-aminocarbonyl-phenyl)-3H-purin-2-on

Hergestellt durch partielle Verseifung von 8-(2-Methoxy-4-cyan-phenyl)-3H-purin-2-on mit konzentrierter Schwefelsäure bei Raumtemperatur.
Schmelzpunkt: über 330°C

| | | | | | | |
|---|---|---|---|---|---|---|
| Ber.: | C | 54,73 | H | 3,88 | N | 24,55 |
| Gef.: | | 54,48 | | 4,12 | | 24,36 |

Beispiel 64

8-(2-Methoxy-4-methylsulfinyl-phenyl)-1H-purin-6-on

Hergestellt analog Beispiel 2 aus 8-(2-Methoxy-4-methylmercapto-phenyl)-1H-purin-6-on.
Schmelzpunkt: 273-276°C.

**Beispiel 65**

**8-(2-Methoxy-4-methylsulfonyl-phenyl)-1H-purin-6-on**

Hergestellt analog Beispiel 2 mit Ameisensäure/Wasserstoffperoxid aus 8-(2-Methoxy-4-methylmercapto-phenyl)-1H-purin-6-on.
Schmelzpunkt: 309-312°C

**Beispiel 66**

**8-(2-Methoxy-4-aminosulfonyl-phenyl)-1H-purin-6-on**

Hergestellt analog Beispiel 28b aus 4-Amino-5-(2-methoxy-4-aminosulfonyl-benzoylamino)-1H-pyrimidin-6-on.
Schmelzpunkt: 294-297°C

**Beispiel 67**

**8-(2-Methoxy-4-methylaminosulfonyl-phenyl)-1H-purin-6-on**

Hergestellt analog Beispiel 28b aus 4-Amino-5-(2-methoxy-4-methylaminosulfonyl-benzoylamino)-1H-pyrimidin-6-on.
Schmelzpunkt: 280-283°C

**Beispiel 68**

**8-(2-Methoxy-4-dimethylaminosulfonyl-phenyl)-1H-purin-6-on**

Hergestellt analog Beispiel 28b aus 4-Amino-5-(2-methoxy-4-dimethylaminosulfonyl-benzoylamino)-1H-pyrimidin-6-on.
Schmelzpunkt: 230°C

## Beispiel 69

### 8-(2-Methoxy-4-aminocarbonyl-phenyl)-1H,3H-purin-2,6-dion

2,3 g 8-(2-Methoxy-4-carboxy-phenyl)-1H,3H-purin-2,6-dion werden in 50 ml Thionylchlorid unter Zusatz eines Tropfens Dimethylformamid eine Stunde zum Rückfluß erhitzt. Überschüssiges Thionylchlorid wird abgezogen und der Rückstand mit einer Lösung von Ammoniak in Dimethylformamid 16 Stunden bei Raumtemperatur gerührt. Man zieht das Dimethylformamid ab, versetzt mit Wasser, säuert mit Eisessig an und filtriert das ausgefallene Produkt ab.

Ausbeute: 1,5 g (63 % der Theorie),

Schmelzpunkt: über 320°C

| | | | | | | |
|---|---|---|---|---|---|---|
| Ber.: | C | 51,83 | H | 3,68 | N | 23,23 |
| Gef.: | | 51,45 | | 3,88 | | 23,12 |

## Beispiel 70

### 8-(2-Methoxy-4-aminocarbonyl-phenyl)-1H-purin-6-on

Hergestellt analog Beispiel 69 aus 8-(2-Methoxy-4-carboxy-phenyl)-1H-purin-6-on. Die Verbindung kristallisiert als Hydrat.

Schmelzpunkt: über 300°C

| | | | | | | |
|---|---|---|---|---|---|---|
| Ber.: | C | 48,59 | H | 4,71 | N | 21,80 |
| Gef.: | | 48,70 | | 4,89 | | 21,60 |

## Beispiel 71

### 8-(2-Methoxy-4-cyan-phenyl)-1H,3H-purin-2,6-dion

Hergestellt durch 24-stündiges Kochen von 0,7 g 8-(2-Methoxy-4-aminocarbonyl-phenyl)-1H,3H-purin-2,6-dion in 10 ml

Phosphoroxychlorid. Die Verbindung kristallisiert als Halbhydrat.
Ausbeute: 0,45 g (68 % der Theorie)
Schmelzpunkt: über 310°C

| | | | | | | |
|---|---|---|---|---|---|---|
| Ber.: | C | 53,48 | H | 3,44 | N | 23,99 |
| Gef.: | | 53,60 | | 3,12 | | 23,12 |

Beispiel 72

2-Methoxy-8-(2-methoxy-4-chlor-5-methylaminosulfonyl-phenyl)-purin

Hergestellt analog Beispiel 11 aus 2-Methoxy-8-(2-methoxy-4-chlor-5-chlorsulfonyl-phenyl)-purin und wäßriger Methylaminlösung.
Schmelzpunkt: 261-263°C

Beispiel 73

8-(2-Methoxy-4-chlor-5-methylaminosulfonyl-phenyl)-3H-purin-2-on

Hergestellt analog Beispiel 6 aus 2-Methoxy-8-(2-methoxy-4-chlor-5-methylaminosulfonyl-phenyl)-purin.
Schmelzpunkt: 270-272°C

Beispiel 74

8-(2-Methoxy-5-methylmercapto-phenyl)-1H,3H-purin-2,6-dion

Hergestellt analog Beispiel 28b aus 4-Amino-5-(2-methoxy-5-methylmercapto-benzoylamino)-1H,3H-pyrimidin-2,6-dion.
Schmelzpunkt: 301-303°C

Beispiel 75

8-(2-Methoxy-5-methylsulfinyl-phenyl)-1H,3H-purin-2,6-dion

Hergestellt analog Beispiel 2 aus 8-(2-Methoxy-5-methylmercapto-phenyl)-1H,3H-purin-2,6-dion.
Schmelzpunkt: 272-275°C

Beispiel 76

8-(2-Methoxy-5-methylsulfonyl-phenyl)-1H,3H-purin-2,6-dion

Hergestellt analog Beispiel 65 aus 8-(2-Methoxy-5-methylmercapto-phenyl)-1H,3H-purin-2,6-dion.
Schmelzpunkt: über 320°C

| | | | | | | | | |
|---|---|---|---|---|---|---|---|---|
| Ber.: | C | 46,43 | H | 3,59 | N | 16,66 | S | 9,53 |
| Gef.: | | 45,94 | | 3,72 | | 16,53 | | 8,70 |

Beispiel 77

2-Methoxy-8-(2-hydroxy-5-methoxy-phenyl)-purin

Hergestellt analog Beispiel 40 aus 2-Methoxy-8-(2-benzyloxy-5-methoxy-phenyl)-purin. Die Verbindung kristallisiert als Hydrat.
Schmelzpunkt: über 300°C

| | | | | | | |
|---|---|---|---|---|---|---|
| Ber.: | C | 52,78 | H | 4,86 | N | 19,30 |
| Gef.: | | 52,66 | | 4,59 | | 19,40 |

## Beispiel 78

### 2-Methoxy-8-(2-propyloxy-5-methoxy-phenyl)-purin

Hergestellt analog Beispiel 28b aus 2-Methoxy-4-amino-5-(2-propyloxy-5-methoxy-benzoylamino)-pyrimidin.
Schmelzpunkt: 140-142°C

## Beispiel 79

### 8-(2-Propyloxy-5-methoxy-phenyl)-3H-purin-2-on

Hergestellt analog Beispiel 6 aus 2-Methoxy-8-(2-propyloxy-5-methoxy-phenyl)-purin.
Schmelzpunkt: 236-238°C

## Beispiel 80

### 8-(2-Ethoxy-4-methoxy-phenyl)-3H-purin-2-on

Hergestellt analog Beispiel 6 aus 2-Methoxy-8-(2-ethoxy-4-methoxy-phenyl)-purin.
Schmelzpunkt: 250-252°C

## Beispiel 81

### 2-Methoxy-8-(2-ethoxy-4-methoxy-phenyl)-purin

Hergestellt analog Beispiel 28b aus 2-Methoxy-4-amino-5-(2-ethoxy-4-methoxy-benzoylamino)-pyrimidin.
Schmelzpunkt: 168-170°C

## Beispiel 82

8-(2-Ethoxy-4-hydroxy-phenyl)-3H-purin-2-on

Hergestellt analog Beispiel 6 aus 2-Methoxy-8-(2-ethoxy-4-hydroxy-phenyl)-purin.
Schmelzpunkt: 280-283°C

## Beispiel 83

2-Methoxy-8-(2-ethoxy-4-hydroxy-phenyl)-purin

Hergestellt analog Beispiel 40 aus 2-Methoxy-8-(2-ethoxy-4-benzyloxy-phenyl)-purin.
Schmelzpunkt: 185-188°C

## Beispiel 84

2-Benzyloxy-8-(2,4-dimethoxy-phenyl)-purin

Hergestellt analog Beispiel 28b aus 2-Benzyloxy-4-amino-5-(2,4-dimethoxy-benzoylamino)-pyrimidin.
Schmelzpunkt: 252-255°C

## Beispiel 85

2-Methoxy-8-(2-ethoxy-5-methoxy-phenyl)-purin

Hergestellt analog Beispiel 28b aus 2-Methoxy-4-amino-5-(2-ethoxy-5-methoxy-benzoylamino)-pyrimidin.
Schmelzpunkt: 165-168°C

0149200

Beispiel 86

8-(2-Ethoxy-5-methoxy-phenyl)-3H-purin-2-on

Hergestellt analog Beispiel 6 aus 2-Methoxy-8-(2-ethoxy-5-methoxy-phenyl)-purin.
Schmelzpunkt: 220-225°C

Beispiel 87

8-(2-Propyloxy-4-hydroxy-phenyl)-3H-purin-2-on

Hergestellt analog Beispiel 6 aus 2-Methoxy-8-(2-propyloxy-4-hydroxy-phenyl)-purin.
Schmelzpunkt: 260-263°C

Beispiel 88

2-Methoxy-8-(2-methoxy-4-benzyloxy-phenyl)-purin

Hergestellt analog Beispiel 28b aus 2-Methoxy-4-amino-5-(2-methoxy-4-benzyloxy-benzoylamino)-pyrimidin.
Schmelzpunkt: 156-158°C

Beispiel 89

2-Methoxy-8-(2-methoxy-4-hydroxy-phenyl)-purin

Hergestellt analog Beispiel 40 aus 2-Methoxy-8-(2-methoxy-4-benzyloxy-phenyl)-purin.
Schmelzpunkt: 240-242°C

Beispiel 90

1,3-Dimethyl-8-(2-methoxy-4-methylaminosulfonyl-phenyl)-1H,3H-purin-2,6-dion

Hergestellt analog Beispiel 72 aus 1,3-Dimethyl-8-(2-methoxy-4-chlorsulfonyl-phenyl)-1H,3H-purin-2,6-dion.
Schmelzpunkt: 310-311°C

Beispiel 91

1,3-Dimethyl-8-(2-methoxy-4-dimethylaminosulfonyl-phenyl)-1H,3H-purin-2,6-dion

Hergestellt analog Beispiel 11 aus 1,3-Dimethyl-8-(2-methoxy-4-chlorsulfonyl-phenyl)-1H,3H-purin-2,6-dion und Dimethylamin.
Schmelzpunkt: 274-276°C

Beispiel 92

2-Methoxy-8-(2-methoxy-4-chlor-phenyl)-purin

Hergestellt analog Beispiel 28b aus 2-Methoxy-4-amino-5-(2-methoxy-4-chlor-benzoylamino)-pyrimidin.
Schmelzpunkt: 280-282°C

Beispiel 93

1,3-Dimethyl-8-(2-methoxy-4-benzyloxy-phenyl)-1H,3H-purin-2,6-dion

Hergestellt analog Beispiel 14 aus 1,3-Dimethyl-4,5-di-

amino-1H,3H-pyrimidin-2,6-dion und 2-Methoxy-4-benzyloxy-benzoesäure.
Schmelzpunkt: 210-213°C

Beispiel 94

1,3-Dimethyl-8-(2-methoxy-4-hydroxy-phenyl)-1H,3H-purin-2,6-dion

Hergestellt analog Beispiel 40 aus 1,3-Dimethyl-8-(2-methoxy-4-benzyloxy-phenyl)-1H,3H-purin-2,6-dion.
Schmelzpunkt: über 310°C

| | | | | | | |
|---|---|---|---|---|---|---|
| Ber.: | C | 56,00 | H | 4,66 | N | 18,50 |
| Gef.: | | 55,63 | | 4,67 | | 18,53 |

Beispiel 95

1,3-Dimethyl-8-(2-methoxy-4-aminosulfonyl-phenyl)-1H,3H-purin-2,6-dion

Hergestellt analog Beispiel 11 aus 1,3-Dimethyl-8-(2-methoxy-4-chlorsulfonyl-phenyl)-1H,3H-purin-2,6-dion.
Schmelzpunkt: 304-306°C

Beispiel 96

1,3-Dimethyl-8-(2-methoxy-4-methylsulfonyl-phenyl)-1H,3H-purin-2,6-dion

Hergestellt analog Beispiel 65 aus 1,3-Dimethyl-8-(2-methoxy-4-methylmercapto-phenyl)-1H,3H-purin-2,6-dion.
Schmelzpunkt: 294-297°C

Beispiel 97

2-Methoxy-8-(2,4,5-trimethoxy-phenyl)-purin

Hergestellt analog Beispiel 28b aus 2-Methoxy-4-amino-5-(2,4,5-trimethoxy-benzoylamino)-pyrimidin.
Schmelzpunkt: 126-128°C

Beispiel 98

2-Methoxy-8-(2,4-dimethoxy-phenyl)-purin

Hergestellt analog Beispiel 28b aus 2-Methoxy-4-amino-5-(2,4-dimethoxy-benzoylamino)-pyrimidin.
Schmelzpunkt: 211-213°C

Beispiel 99

2-Benzyloxy-8-(2-methoxy-4-methylmercapto-phenyl)-purin

Hergestellt analog Beispiel 28b aus 2-Benzyloxy-4-amino-5-(2-methoxy-4-methylmercapto-benzoylamino)-pyrimidin.
Schmelzpunkt: 278-281°C

Beispiel 100

6-Chlor-8-(2-methoxy-5-methylmercapto-phenyl)-purin

Hergestellt analog Beispiel 14 aus 4,5-Diamino-6-chlor-pyrimidin und 2-Methoxy-5-methylmercapto-benzoesäure.
Schmelzpunkt: 172-175°C

Beispiel 101

6-Chlor-8-(2,4-dimethoxy-5-brom-phenyl)-purin

Hergestellt analog Beispiel 14 aus 4,5-Diamino-6-chlor-pyrimidin und 2,4-Dimethoxy-5-brom-benzoesäure.
Schmelzpunkt: 260-263°C

Beispiel 102

6-Chlor-8-(2-methoxy-4-benzyloxy-phenyl)-purin

Hergestellt analog Beispiel 28b aus 4-Amino-5-(2-methoxy-4-benzyloxy-benzoylamino)-6-chlor-pyrimidin.
Schmelzpunkt: 203-205°C

Beispiel 103

2-(2-Methoxy-4-methoxycarbonyl-phenyl)-5H-imidazo[4,5-c]-pyridin-4-on-hydrochlorid

Hergestellt analog Beispiel 1 aus 2-Methoxy-3,4-diaminopyridin und 2-Methoxy-4-cyan-benzoesäure. Das erhaltene Produktgemisch wird mit Dichlorethan/Ethanol 3:1 digeriert. Man filtiert das ungelöste Material ab, dampft die Lösung ein und kocht mit methanolischer Salzsäure. Beim Abkühlen kristallisiert das Produkt als Hydrat aus.
Schmelzpunkt: 224-226°C (Zersetzung)

Beispiel 104

4-Methoxy-2-(2-methoxy-4-aminocarbonyl-phenyl)-imidazo-[4,5-c]pyridin

Hergestellt wie in Beispiel 103. Das angefallene unlösliche Produkt wird über Kieselgel (Elutionsmittel: Dichlorethan/-Ethanol = 100:0 bis 95:5) gereinigt.
Schmelzpunkt des Halbhydrochlorids: 274-276°C

Beispiel 105

2-(2-Methoxy-4-aminosulfonyl-phenyl)-5H-imidazo[4,5-d]pyridazin-4-on

Hergestellt analog Beispiel 11 aus 2-(2-Methoxy-4-chlorsulfonyl-phenyl)-5H-imidazo[4,5-d]pyridazin-4-on (erhalten aus 2-(2-Methoxy-4-amino-phenyl)-5H-imidazo[4,5-d]pyridazin-4-on durch Diazotieren in konzentrierter Salzsäure und Behandeln mit einer Lösung von Schwefeldioxid in Eisessig in Gegenwart von Kupfer(II)chlorid).
Schmelzpunkt: 303-304°C (Zersetzung)

Beispiel 106

2-(2-Methoxy-4-methylaminosulfonyl-phenyl)-5H-imidazo[4,5-d]-pyridazin-4-on

Hergestellt analog Beispiel 11 aus 2-(2-Methoxy-4-chlorsulfonyl-phenyl)-5H-imidazo[4,5-d]pyridazin-4-on und wäßriger Methylaminlösung.
Schmelzpunkt: 306-311°C (Zersetzung)

Beispiel 107

2-(2-Methoxy-4-dimethylaminosulfonyl-phenyl)-5H-imidazo-[4,5-d]pyridazin-4-on

Hergestellt analog Beispiel 1 aus 2-Methoxy-4-dimethylamino-sulfonyl-benzoesäure.
Schmelzpunkt: 308-317°C (Zersetzung)

Beispiel 108

2-(2-Methoxy-4-aminocarbonyl-phenyl)-5H-imidazo[4,5-d]pyridazin-4-on

Hergestellt analog Beispiel 1 aus 2-Methoxy-4-cyan-benzoesäure.
Schmelzpunkt: 317-330°C (Zersetzung)

Beispiel 109

2-(2-Hydroxy-4-amino-phenyl)-5H-imidazo[4,5-c]pyridazin-6-on

0,2 g 2-(2-Methoxy-4-amino-phenyl)-6-chlor-imidazo[4,5-c]-pyridazin werden bei 170-190°C 5 Minuten mit geschmolzenem Kaliumhydroxid behandelt. Das Produkt wird über Kieselgel (Elutionsmittel: Methylenchlorid/Ethanol = 100:0 bis 80:20) gereinigt.
Ausbeute: 170 mg (96 % der Theorie),
Schmelzpunkt: über 360°C

Beispiel 110

2-(2-Methoxy-4-amino-phenyl)-6-chlor-imidazo[4,5-c]-pyridazin

Hergestellt analog Beispiel 1 aus 3,4-Diamino-6-chlor-pyridazin und 2-Methoxy-4-amino-benzoesäure.
Schmelzpunkt: 268-270°C (Zersetzung)

Beispiel 111

2-(2-Methoxy-4-acetylamino-phenyl)-5H-imidazo[4,5-d]pyridazin-4-on

Hergestellt analog Beispiel 34 aus 2-(2-Methoxy-4-amino-phenyl)-5H-imidazo[4,5-d]pyridazin-4-on.
Schmelzpunkt: 315-320°C (Zersetzung)

Beispiel 112

2-(2-Methoxy-4-benzyloxy-phenyl)-4-chlor-imidazo[4,5-d]-pyridazin

Hergestellt analog Beispiel 5 aus 4,5-Diamino-2H-pyridazin-3-on und 2-Methoxy-4-benzyloxy-benzoylchlorid.
Schmelzpunkt: sintert bei 172-175°C (Zersetzung)

Analog den vorstehenden Beispielen lassen sich folgende Verbindungen herstellen:

2-(2-Methoxy-4-cyan-phenyl)-6-chlor-5H-imidazo[4,5-c]pyridin-4-on

2-(2-Methoxy-4-aminosulfonyl-phenyl)-5H-imidazo[4,5-c]pyridin-4-on

2-(2-Methoxy-4-methylaminosulfonyl-phenyl)-5H-imidazo[4,5-c]-pyridin-4-on

2-(2-Methoxy-4-chlor-5-aminosulfonyl-phenyl)-5H-imidazo-[4,5-c]pyridin-4-on

2-(2-Methoxy-4-methyl-5-aminosulfonyl-phenyl)-5H-imidazo-[4,5-c]pyridin-4-on

2-(2-Methoxy-4-aminosulfonyl-phenyl)-5H-imidazo[4,5-c]pyridin-6-on

2-(2-Methoxy-4-aminosulfonyl-phenyl)-5H-imidazo[4,5-c]pyridazin-6-on

2-(2-Methoxy-4-dimethylaminosulfonyl-phenyl)-5H-imidazo-[4,5-c]pyridin-6-on

2-(2-Methoxy-4-cyan-phenyl)-5H-imidazo[4,5-c]pyridin-4-on

2-(2-Methoxy-4-methylsulfinyl-phenyl)-7H-imidazo[4,5-e]-1,2,4-triazin-6-on

2-(2-Methoxy-4-methylsulfonyl-phenyl)-7H-imidazo[4,5-e]-1,2,4-triazin-6-on

2-(2-Methoxy-4-cyan-phenyl)-5H-imidazo[4,5-d]pyridazin-4-on

Beispiel A

Tabletten zu 100 mg 8-(2-Methoxy-4-methylmercapto-phenyl)-purin-2-on

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

Beispiel B

Dragées zu 50 mg 8-(2-Methoxy-4-methylmercapto-phenyl)-purin-2-on

Zusammensetzung:
1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

Feuchtsiebung: 1,0 mm

Trockensiebung: 1,0 mm,

Trocknung: 50°C im Umlufttrockenschrank

Granulat und restliche Hilfsstoffe mischen und zu Kernen verpressen.

Kerngewicht: 80 mg

Stempel: 6 mm

Wölbungsradius: 5 mm

Die fertigen Kerne werden auf übliche Weise mit einem Zuckerüberzug im Dragierkessel versehen.

Dragéegewicht: 120 mg

## Beispiel C

**Suppositorien zu 75 mg 8-(2-Methoxy-4-methylmercapto-phenyl)-purin-2-on**

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

### Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 8-(2-Methoxy-4-methylmercapto-phenyl)-purin-2-on

1 Ampulle enthält:

| | |
|---|---|
| Wirksubstanz | 50,0 mg |
| Ethoxylierte Hydroxystearinsäure | 250,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.

Abfüllung: in Ampullen zu 5 ml

Sterilisation: 20 Minuten bei 120°C

Beispiel E

Tropfen zu 100 mg 8-(2-Methoxy-4-methylmercapto-phenyl)-purin-2-on

| | | | |
|---|---|---|---|
| Wirksubstanz | | 1,0 | g |
| p-Oxybenzoesäuremethylester | | 0,035 | g |
| p-Oxybenzoesäurepropylester | | 0,015 | g |
| Anisöl | | 0,05 | g |
| Menthol | | 0,06 | g |
| Saccharin-Natrium | | 1,0 | g |
| Glycerin | | 10,0 | g |
| Ethanol | | 40,0 | g |
| Dest. Wasser | ad | 100,0 | ml |

0149200

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

## Patentansprüche

1. 2-Phenyl-imidazole der allgemeinen Formel

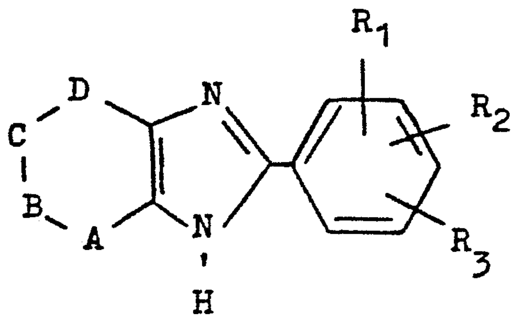

,(I)

in der

A, B, C und D je ein gegebenenfalls durch ein Wasserstoffatom oder eine Alkylgruppe mit 1 bis 3 Kohlenstoffatomen substituiertes Stickstoffatom, ein durch ein Wasserstoffatom, ein Halogenatom, eine Hydroxygruppe, eine Benzyloxygruppe oder eine Alkoxygruppe mit 1 bis 3 Kohlenstoffatomen substituiertes Kohlenstoffatom oder eine Carbonylgruppe, wobei jedoch mindestens einer der Reste A, B, C oder D ein gegebenenfalls durch ein Wasserstoffatom oder eine Alkylgruppe substituiertes Stickstoffatom und ein weiterer der Reste A, B, C oder D ein durch ein Halogenatom, eine Hydroxy-, Benzyloxy- oder Alkoxygruppe substituiertes Kohlenstoffatom oder eine Carbonylgruppe darstellen muß,

$R_1$ eine Nitril-, Aminocarbonyl-, Alkylaminocarbonyl-, Dialkylaminocarbonyl-, Alkoxycarbonyl- oder Alkanoylaminogruppe oder,

wenn A, B, C und D zusammen mit dem Rest des Moleküls kein 8-Phenyl-xanthin darstellen, in dem der Phenylring in 2- und/oder 6-Stellung unsubstituiert ist, auch eine Aminosulfonyl-, Alkylaminosulfonyl- oder Dialkylaminosulfonylgruppe oder,

wenn A ein durch ein Wasserstoffatom oder durch eine Alkylgruppe substituiertes Stickstoffatom darstellt, auch eine Alkylmercapto-, Alkylsulfinyl- oder Alkylsulfonylgruppe mit 1 bis 3 Kohlenstoffatomen in jedem Alkylteil, oder,

wenn A ein gegebenenfalls durch ein Wasserstoffatom oder eine Alkylgruppe substituiertes Stickstoffatom darstellt und der Phenylring in 2-Stellung durch eine Alkoxy- oder Dialkylaminogruppe substituiert ist, auch ein Halogenatom, eine Alkyl-, Nitro-, Carboxy-, Amino-, Alkylamino-, Dialkylamino- oder Benzyloxygruppe oder eine 5-ständige Alkoxygruppe oder auch, wenn die Reste A, B, C und D zusammen mit dem Imidazolring keinen Theophyllinrest darstellen, auch eine 4-ständige Alkoxygruppe, wobei der Alkylteil jeweils 1 bis 3 Kohlenstoffatome enthalten kann, oder,

wenn A ein durch ein Wasserstoffatom oder durch eine Alkylgruppe substituiertes Stickstoffatom und $R_2$ und $R_3$ jeweils kein Wasserstoffatom darstellen, auch ein Halogenatom, eine Nitro-, Benzyloxy-, Hydroxysulfonyl- oder Alkoxygruppe mit 1 bis 3 Kohlenstoffatomen oder,

wenn A und mindestens einer der Reste B und D ein gegebenenfalls durch ein Wasserstoffatom oder eine Alkylgruppe substituiertes Stickstoffatom darstellen, auch ein Halogenatom, eine Alkyl-, Hydroxy-, Benzyloxy-, Alkoxy-, Alkylmercapto-, Alkylsulfinyl-, Alkylsulfonyl-, Hydroxysulfonyl-, Nitro-, Amino-, Alkylamino- oder Dialkylaminogruppe, wobei jeweils der Alkylteil 1 bis 3 Kohlenstoffatome enthalten kann, oder,

wenn B und C jeweils gleichzeitig ein gegebenenfalls durch ein Wasserstoffatom oder eine Alkylgruppe substituiertes Stickstoffatom darstellen, auch ein Halogenatom, eine Alkyl-, Hydroxy-, Benzyloxy-, Alkoxy-, Alkylmercapto-, Alkylsulfinyl-, Alkylsulfonyl-, Hydroxysulfonyl-, Nitro-, Amino-, Alkylamino- oder Dialkylaminogruppe, wobei der Alkylteil jeweils 1 bis 3 Kohlenstoffatome enthalten kann, oder,

wenn A ein Stickstoffatom und C ein durch eine Alkylgruppe substituiertes Stickstoffatom darstellen, auch eine Benzyloxy-, Alkylmercapto-, Alkylsulfinyl- oder Alkylsulfonylgruppe oder, wenn die Reste A, B, C und D zusammen mit dem Imidazolring keinen Xanthinrest darstellen, auch eine Alkyl-, Hydroxy-, Alkoxy-, Nitro-, Amino-, Alkylamino- oder Dialkylaminogruppe, wobei jeweils der Alkylteil 1 bis 3 Kohlenstoffatome enthalten kann, oder

wenn A ein Stickstoffatom und B ein durch eine Alkoxygruppe substituiertes Kohlenstoffatom darstellen, auch ein Halogenatom, eine Alkyl-, Nitro-, Amino-, Alkylamino-, Dialkylamino-, Hydroxy-, Alkoxy-, Benzyloxy-, Alkylmercapto-, Alkylsulfinyl-, Alkylsulfonyl-, Hydroxysulfonyl- oder Carboxygruppe, wobei jeweils jeder Alkylteil 1 bis 3 Kohlenstoffatome enthalten kann,

$R_2$ und $R_3$, die gleich oder verschieden sein können, Wasserstoffatome, Halogenatome, Alkyl-, Hydroxy-, Alkoxy-, Benzyloxy-, Alkylmercapto-, Alkylsulfinyl-, Alkylsulfonyl-, Nitro-, Amino-, Alkylamino-, Dialkylamino-, Alkanoylamino-, Aminosulfonyl-, Alkylaminosulfonyl-, Dialkylaminosulfonyl-, Nitril-, Carboxy-, Alkoxycarbonyl-, Aminocarbonyl-, Alkylaminocarbonyl- oder Dialkylaminocarbonylgruppen, wobei jeweils der Alkylteil 1 bis 3 Kohlenstoffatome enthalten kann, bedeuten deren Tautomere und deren Säureadditionssalze.

2. 2-Phenylimidazole der allgemeinen Formel I gemäß Anspruch 1, in der A, B, C und D je ein gegebenenfalls durch ein Wasserstoffatom oder eine Methylgruppe substituiertes Stickstoffatom, ein durch ein Wasserstoff-, Chlor- oder Bromatom, eine Hydroxy-, Methoxy- oder Benzyloxygruppe substituiertes Kohlenstoffatom oder eine Carbonylgruppe, wobei jedoch mindestens einer der Reste A, B , C oder D ein gegebenenfalls durch ein Wasserstoffatom oder eine Methylgruppe substituiertes Stickstoffatom und ein weiterer der Reste A, B, C oder D ein durch ein Chloratom, eine Hydroxy-, Methoxy- oder Benzyloxygruppe substituiertes Kohlenstoffatom oder eine Carbonylgruppe darstellen muß,

$R_1$ eine Nitril-, Aminocarbonyl-, Methylaminocarbonyl-, Dimethylaminocarbonyl-, Methoxycarbonyl- oder Acetylaminogruppe oder,

wenn A, B, C und D zusammen mit dem Rest des Moleküls kein 8-Phenyl-xanthin darstellen, in dem der Phenylring in 2- und/oder 6-Stellung unsubstituiert ist, auch eine Aminosulfonyl-, Methylaminosulfonyl- oder Dimethylaminosulfonylgruppe oder,

wenn A ein durch ein Wasserstoffatom oder eine Methylgruppe substituiertes Stickstoffatom darstellt, auch eine Methylmercapto-, Methylsulfinyl- oder Methylsulfonylgruppe oder,

wenn A ein gegebenenfalls durch ein Wasserstoffatom oder eine Methylgruppe substituiertes Stickstoffatom darstellt und der Phenylring in 2-Stellung durch eine Methoxy-, Ethoxy-, Propoxy- oder Dimethylaminogruppe substituiert ist, auch ein Chlor- oder Bromatom, eine Methyl-, Nitro-, Amino-, Methylamino-, Dimethylamino- oder Benzyloxygruppe oder eine 5-ständige Methoxygruppe oder, wenn die Reste A, B, C und D zusammen mit dem Imidazolring keinen Theophyllinrest darstellen, auch eine 4-ständige Methoxygruppe oder,

wenn A ein durch ein Wasserstoffatom oder eine Methylgruppe substituiertes Stickstoffatom und $R_2$ und $R_3$ jeweils kein Wasserstoffatom darstellen, auch ein Chlor- oder Bromatom, eine Nitro-, Hydroxysulfonyl-, Methoxy-, Ethoxy-, Propoxy- oder Benzyloxygruppe oder,

wenn A und mindestens einer der Reste B oder D ein gegebenenfalls durch ein Wasserstoffatom oder eine Methylgruppe substituiertes Stickstoffatom darstellen, auch ein Chloratom, eine Methyl-, Hydroxy-, Benzyloxy-, Methoxy-, Ethoxy-, Propoxy-, Methylmercapto-, Methylsulfinyl-, Methylsulfonyl-, Hydroxysulfonyl-, Amino-, Methylamino- oder Dimethylaminogruppe oder,

wenn B und C jeweils gleichzeitig ein gegebenenfalls durch ein Wasserstoffatom oder eine Methylgruppe substituiertes Stickstoffatom darstellen, auch ein Chloratom, eine Methyl-, Hydroxy-, Methoxy-, Ethoxy-, Propoxy-, Benzyloxy-, Methylmercapto-, Methylsulfinyl-, Methylsulfonyl-, Hydroxysulfonyl-, Nitro-, Amino-, Methylamino- oder Dimethylaminogruppe oder,

wenn A ein Stickstoffatom und C ein durch eine Methylgruppe substituiertes Stickstoffatom darstellen, auch eine Methylmercapto-, Methylsulfinyl- oder Methylsulfonylgruppe oder, wenn die Reste A, B, C und D zusammen mit dem Imidazolring keinen Xanthinrest darstellen, auch eine Methyl-, Hydroxy-, Methoxy-, Ethoxy-, Propoxy-, Nitro-, Amino-, Methylamino- oder Dimethylaminogruppe oder,

wenn A ein Stickstoffatom und B ein durch eine Methoxygruppe substituiertes Kohlenstoffatom darstellen, auch ein Chlor- oder Bromatom, eine Methyl-, Nitro-, Amino-, Methylamino-, Dimethylamino-, Hydroxy-, Methoxy-, Ethoxy-, Propoxy-, Benzyloxy-, Methylmercapto-, Methylsulfinyl-, Methylsulfonyl-, Hydroxysulfonyl- oder Carboxylgruppe,

$R_2$ ein Wasserstoff-, Chlor- oder Bromatom, eine Methyl-, Hydroxy-, Methoxy-, Ethoxy-, Benzyloxy-, Methylmercapto-, Methylsulfinyl-, Methylsulfonyl-, Nitro-, Amino-, Methylamino-, Dimethylamino-, Acetylamino-, Aminosulfonyl-, Methylaminosulfonyl-, Dimethylaminosulfonyl-, Methoxycarbonyl-, Aminocarbonyl-, Methyl- aminocarbonyl- oder Dimethylaminocarbonylgruppe und

$R_3$ ein Wasserstoffatom, eine Dimethylamino-, Hydroxy-, Methoxy-, Ethoxy- oder Propoxygruppe bedeuten, deren Tautomere und deren Säureadditionssalze.

3. 2-Phenyl-imidazole der allgemeinen Formel I gemäß Anspruch 1, in der
A, B, C, D und $R_1$ wie im Anspruch 2 definiert sind, wobei
$R_1$ in 4- oder 5-Stellung steht,
$R_3$ in 2-Stellung mit Ausnahme des Wasserstoffatoms die für $R_3$ im Anspruch 2 erwähnten Bedeutungen besitzt und
$R_2$ ein Wasserstoff-, Chlor- oder Bromatom, eine Nitro-, Aminosulfonyl- oder Methylaminosulfonylgruppe darstellt, deren Tautomere und deren Säureadditionssalze.

4. 2-Phenyl-imidazole der allgemeinen Formel I gemäß Anspruch 1, in der
A, B, C, D, $R_1$ und $R_3$ wie im Anspruch 2 definiert sind und
$R_2$ ein Wasserstoffatom darstellt, deren Tautomeren und deren Säureadditionssalze.

5. 2-Phenyl-imidazole der allgemeinen Formel I gemäß Anspruch 1, in der
A, B, C und D wie im Anspruch 2 definiert sind,
$R_1$ in 4-Stellung eine Benzyloxy-, Methylmercapto-, Methylsulfinyl-, Methylsulfonyl-, Aminosulfonyl-, Methylaminosulfonyl- oder Dimethylaminosulfonylgruppe,

$R_3$ in 2-Stellung eine Methoxygruppe und $R_2$ ein Wasserstoffatom bedeuten, sowie deren Tautomere und deren Säureadditionssalze, insbesondere deren physiologisch verträglichen Säureadditionssalze mit anorganischen oder organischen Säuren.

6. 2-(2-Methoxy-4-methylsulfonyl-phenyl)-5H-imidazo[4,5-d]-pyridazin-4-on, dessen Tautomere und dessen Säureadditionssalze.

7. Physiologisch verträgliche Säureadditionssalze mit anorganischen oder organischen Säuren gemäß den Ansprüchen 1 bis 6.

8. Arzneimittel, enthaltend eine Verbindung gemäß den Ansprüchen 1 bis 6 oder ein physiologisch verträgliches Säureadditionssalz gemäß Anspruch 7 neben einem oder mehreren inerten Trägerstoffen und/oder Verdünnungsmitteln.

9. Verwendung einer Verbindung gemäß den Ansprüchen 1 bis 6 oder eines physiologisch verträglichen Säureadditionssalzes gemäß Anspruch 7 zur Behandlung von Herzinsuffizienzen.

10. Verfahren zur Herstellung eines Arzneimittels, dadurch gekennzeichnet, daß auf nichtchemischem Wege eine Verbindung gemäß den Ansprüchen 1-6 oder ein physiologisch verträgliches Säureadditionssalz gemäß Anspruch 7 in einen oder mehreren inerten Trägerstoffen und/oder Verdünnungmitteln eingearbeitet wird.

11. Verfahren zur Herstellung von neuen 2-Phenyl-imidazolen gemäß den Ansprüchen 1-7, dadurch gekennzeichnet, daß

a) eine gegebenenfalls im Reaktionsgemisch hergestellte Verbindung der allgemeinen Formel

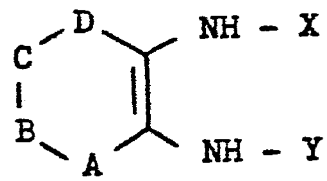

,(II)

in der A bis D wie im Anspruch 1 definiert sind, einer der Reste X oder Y ein Wasserstoffatom und der andere der beiden Reste X und Y oder beide Reste X und Y eine Gruppe der Formel

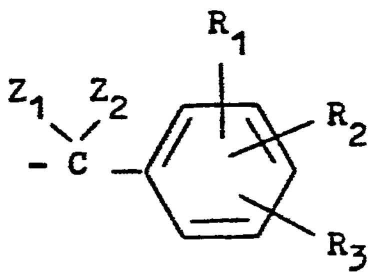

darstellen, in der

$R_1$ bis $R_3$ wie im Anspruch 1 definiert sind, $Z_1$ und $Z_2$, die gleich oder verschieden sein können, gegebenenfalls substituierte Aminogruppe oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel I, in der mindestens einer der Reste $R_1$, $R_2$ oder $R_3$ eine Alkylsulfinyl- oder Alkylsulfonylgruppe darstellt, eine Verbindung der allgemeinen Formel

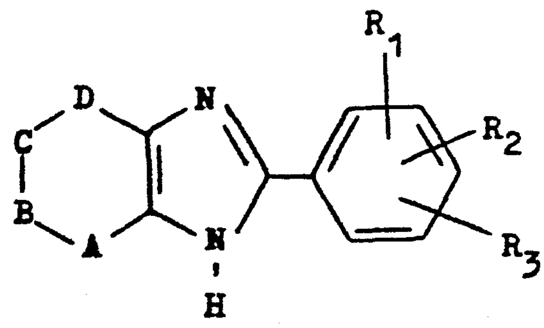

,(III)

in der
A bis D und $R_1$ bis $R_3$ wie im Anspruch 1 definiert sind, wobei jedoch mindestens einer der Reste $R_1$, $R_2$ oder $R_3$ eine Alkylmercapto- oder Alkylsulfinylgruppe mit jeweils 1 bis 3 Kohlenstoffatomen in jedem Alkylteil darstellen muß, oxidiert wird oder

c) zur Herstellung von Verbindungen der allgemeinen Formel I, in der mindestens einer der Reste $R_1$, $R_2$ oder $R_3$ eine Hydroxysulfonyl-, Aminosulfonyl-, Alkylaminosulfonyl- oder Dialkylaminosulfonylgruppe darstellt, eine gegebenenfalls im Reaktionsgemisch gebildete Verbindung der allgemeinen Formel

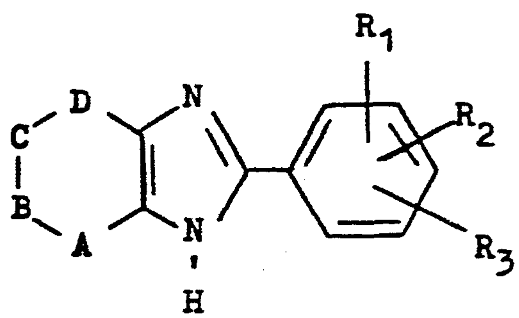

,(IV)

in der
A bis D und $R_1$ bis $R_3$ wie im Anspruch 1 definiert sind, wobei jedoch mindestens einer der Reste $R_1$, $R_2$ oder $R_3$ eine Gruppe der Formel $USO_2$- darstellen muß, in der U eine nukleophile Austrittsgruppe wie ein Halogenatom darstellt, mit einer Verbindung der allgemeinen Formel

$$H - V \quad ,(V)$$

in der
V eine Hydroxygruppe oder eine gegebenenfalls durch eine oder zwei Alkylgruppen mit jeweils 1 bis 3 Kohlenstoffatomen substituierte Aminogruppe darstellt, umgesetzt wird oder

d) zur Herstellung von Verbindungen der allgemeinen Formel I, in der mindestens einer der Reste $R_1$, $R_2$ oder $R_3$ eine durch eine Amino-, Alkylamino- oder Dialkylaminogruppe substituierte Carbonylgruppe darstellt, eine Verbindung der allgemeinen Formel

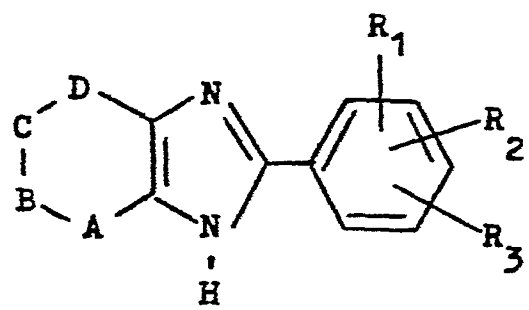
,(VI)

in der
A bis D und $R_1$ bis $R_3$ wie im Anspruch 1 definiert sind, wobei jedoch mindestens einer der Reste $R_1$, $R_2$ oder $R_3$ die W-CO-Gruppe, in der
W eine Hydroxygruppe oder eine nukleophile Austrittsgruppe darstellt, darstellen muß oder eines reaktionsfähigen Derivates hiervon mit einem Amin der allgemeinen Formel

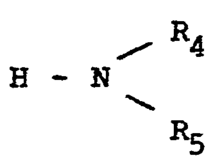
(VII)

in der
$R_4$ und $R_5$, die gleich oder verschieden sein können, Wasserstoffatome oder Alkylgruppen mit 1 bis 3 Kohlenstoffatome darstellen, oder mit einem reaktionsfähigen Derivat hiervon, falls W die Hydroxygruppe darstellt, umgesetzt wird oder

e) zur Herstellung von Verbindungen der allgemeinen Formel I, in der mindestens einer der Reste A, B, C oder D ein durch eine Hydroxy-, Alkoxy- oder Phenylalkoxygruppe substituiertes Kohlenstoffatom oder die Carbonylgruppe darstellt, eine Verbindung der allgemeinen Formel

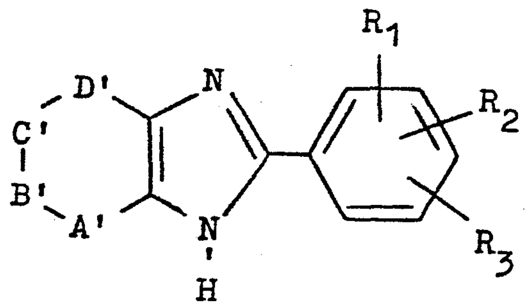

,(VIII)

in der

einer der Reste A', B', C' oder D' ein durch ein Halogenatom substituiertes Kohlenstoffatom darstellt und die übrigen der Reste A' bis D' die für A bis D im Anspruch 1 erwähnten Bedeutungen besitzen, mit einer Verbindung der allgemeinen Formel

$$H - R_6 \quad ,(IX)$$

in der

$R_6$ eine Hydroxy-, Alkoxy- oder Phenylalkoxygruppe darstellt, wobei der Alkylteil jeweils 1 bis 3 Kohlenstoffatome enthalten kann, umgesetzt wird oder

f) zur Herstellung von Verbindungen der allgemeinen Formel I, in der mindestens einer der Reste A, B, C oder D ein durch eine Hydroxygruppe substituiertes Kohlenstoffatom oder die Carbonylgruppe darstellt, ein N-Oxid der allgemeinen Formel

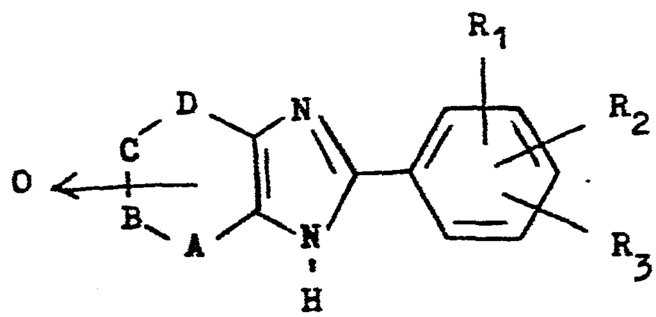

,(X)

in der

A bis D und $R_1$ bis $R_3$ wie im Anspruch 1 definiert sind, umgelagert und gegebenenfalls anschließend hydrolysiert wird oder

g) zur Herstellung von Verbindungen der allgemeinen Formel I, in der mindestens einer der Reste A, B, C oder D ein durch eine Hydroxygruppe substituiertes Kohlenstoffatom oder die Carbonylgruppe und/oder mindestens einer der Reste $R_1$, $R_2$ oder $R_3$ eine Hydroxygruppe darstellen, eine Verbindung der allgemeinen Formel

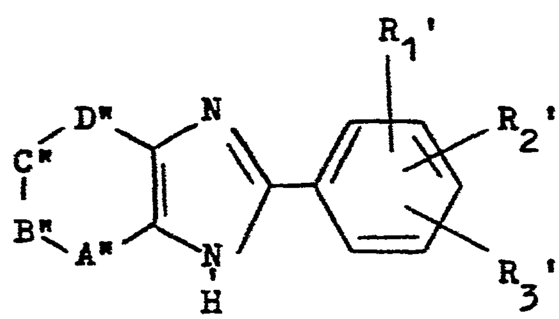

,(XI)

in der

einer der Reste A", B", C" oder D" ein durch eine Benzyloxygruppe substituiertes Kohlenstoffatom darstellt und die übrigen der Reste A" bis D" die für A bis D im Anspruch 1 erwähnten Bedeutungen besitzen, und/oder mindestens einer der Reste $R_1'$, $R_2'$ oder $R_3'$ eine Benzyloxygruppe darstellt und die übrigen der Reste $R_1'$ bis $R_3'$ die für $R_1$ bis $R_3$ im Anspruch 1 erwähnten Bedeutungen besitzen, entbenzyliert wird oder

h) zur Herstellung von Verbindungen der allgemeinen Formel I, in der mindestens einer der Reste $R_1$ bis $R_3$ eine Aminogruppe darstellt, eine Verbindung der allgemeinen Formel

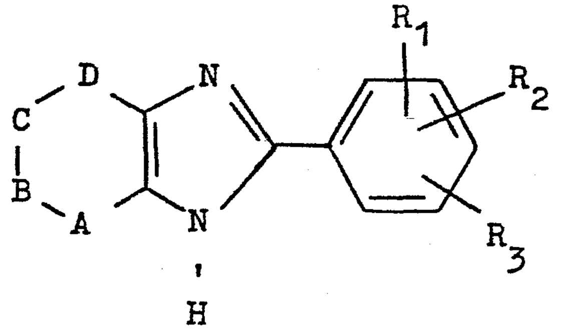

,(XII)

in der
A bis D wie im Anspruch 1 definiert sind,
mindestens einer der Reste $R_1$, $R_2$ oder $R_3$ eine Gruppe der Formel

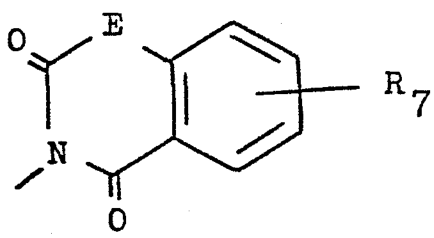

darstellt, wobei

E eine Bindung oder eine gegebenenfalls durch Alkylgruppen mit 1 bis 3 Kohlenstoffatomen substituierte Methylengruppe und
$R_7$ ein Wasserstoffatom oder eine Nitrogruppe bedeuten, und

der andere der Reste $R_1$ bis $R_3$ die für $R_1$ bis $R_3$ im Anspruch 1 erwähnten Bedeutungen besitzt, mit Hydrazin umgesetzt wird

und gewünschtenfalls eine so erhaltene Verbindung der allgemeinen Formel I, in der mindestens einer der Reste A, B, C oder D eine Alkoxymethingruppe und/oder mindestens einer der Reste $R_1$, $R_2$ oder $R_3$ eine Cyangruppe darstellt, mittels Hydrolyse und/oder Alkoholyse in eine entsprechende Verbindung der allgemeinen Formel I, in der mindestens einer der

Reste A, B, C oder D eine Carbonyl- oder Hydroxymethingruppe und/oder mindestens einer der Reste $R_1$, $R_2$ oder $R_3$ eine Aminocarbonyl- oder Alkoxycarbonylgruppe darstellt, übergeführt wird und/oder

eine so erhaltene Verbindung der allgemeinen Formel I, in der mindestens einer der Reste $R_1$, $R_2$ oder $R_3$ eine Nitrogruppe darstellt, mittels Reduktion in eine entsprechende Verbindung der allgemeinen Formel I, in der mindestens einer der Reste $R_1$, $R_2$ oder $R_3$ eine Aminogruppe darstellt, übergeführt wird und/oder

eine so erhaltene Verbindung der allgemeinen Formel I, in der mindestens einer der Reste $R_1$, $R_2$ oder $R_3$ eine Aminogruppe darstellt, mittels Alkanoylierung in eine entsprechende Verbindung der allgemeinen Formel I, in der mindestens einer der Reste $R_1$, $R_2$ oder $R_3$ eine Alkanoylaminogruppe darstellt, übergeführt wird und/oder

eine so erhaltene Verbindung der allgemeinen Formel I, in der mindestens einer der Reste $R_1$, $R_2$ oder $R_3$ eine Aminogruppe darstellt, über ihr Diazoniumsalz in eine entsprechende Verbindung der allgemeinen Formel I, in der einer der Reste $R_1$, $R_2$ oder $R_3$ ein Halogenatom, eine Hydroxy- oder Cyangruppe darstellt, übergeführt werden und/oder

eine so erhaltene Verbindung der allgemeinen Formel I, in der einer der Reste $R_1$, $R_2$ oder $R_3$ eine Aminocarbonylgruppe darstellt, mittels Dehydratisierung in eine entsprechende Verbindung der allgemeinen Formel I, in der $R_1$ eine Cyangruppe darstellt, übergeführt wird und/oder

eine so erhaltene Verbindung der allgemeinen Formel I in ihr Säureadditionssalz, insbesondere in ihr physiologisch verträgliches Säureadditionssalz mit einer anorganischen oder organischen Säure übergeführt wird.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0149200
Nummer der Anmeldung

EP 84116009.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. X 4) |
|---|---|---|---|
| X | EP - A2 - 0 093 593 (ELI LILLY AND COMPANY)<br>* Ansprüche 1 (insbesonders Zeilen 24, 25), 9-11 *<br>-- | 1,2,7, 8,10, 11 | C 07 D 487/04<br>C 07 D 471/04<br>C 07 D 473/00<br>A 61 K 31/395<br>A 61 K 31/415<br>A 61 K 31/44<br>A 61 K 31/52<br>A 61 K 31/50 |
| A | EP - A1 - 0 049 407 (DR. KARL THOMAE GMBH)<br>* Ansprüche 1,4 *<br>-- | 1-5,7, 8,10, 11 | |
| A | EP - A1 - 0 092 398 (WARNER-LAMBERT COMP.)<br>* Ansprüche 1,9,10; Seite 16 *<br>-- | 1-5,7, 8,10, 11 | |
| D,A | EP - A1 - 0 079 083 (THE WELLCOME FOUNDATION LIMITED)<br>* Ansprüche 1,10,11 *<br>-- | 1-5,7, 8,10, 11 | **RECHERCHIERTE SACHGEBIETE (Int. Cl. X 4)**<br>C 07 D 487/00<br>C 07 D 471/00<br>C 07 D 473/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8,10,11
Unvollständig recherchierte Patentansprüche: –
Nicht recherchierte Patentansprüche: 9
Grund für die Beschränkung der Recherche:

Nichtpatentliteratur gemäß Art. 52(4) EPÜ
Behandlung des menschlichen oder tierischen Körpers durch Therapie

| Recherchenort | Abschlußdatum der Recherche | Prüfer |
|---|---|---|
| WIEN | 22-03-1985 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

0149200

Nummer der Anmeldung

Europäisches Patentamt

# EUROPÄISCHER TEILRECHERCHENBERICHT

EP 84116009.6

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| A | DE - A - 2 305 339 (DR. KARL THOMAE GMBH)<br>* Ansprüche 1,9,10 *<br>-- | 1-5,7, 8,10, 11 | |
| A | DE - A - 1 670 940 (FISONS PEST CONTROL LTD.)<br>* Ansprüche 1,24 *<br>-- | 1-5,7, 11 | |
| A | EP - A2 - 0 093 951 (MERCK PATENT GESELLSCHAFT MIT BESCHRÄNKTER HAFTUNGE)<br>* Ansprüche 1,3-5 *<br>---- | 1-7,8, 10,11 | RECHERCHIERTE SACHGEBIETE (Int. Cl. 4) |